# EUROPEAN PATENT SPECIFICATION

(11) **EP 1 646 363 B1**
(45) Date of publication and mention of the grant of the patent: **16.10.2013**
(21) Application number: 04740949.5
(22) Date of filing: 09.07.2004
(51) Int. Cl.: A61K 9/00

(54) **INTRAVAGINAL DRUG DELIVERY DEVICES**
INTRAVAGINALE ARZNEIMITTELABGABEVORRICHTUNGEN
DISPOSITIFS D'ADMINISTRATION DE MEDICAMENTS PAR VOIE INTRAVAGINALE

(30) Priority: 10.07.2003 IE 20030515
(43) Date of publication of application: 19.04.2006
(73) Proprietor: Warner Chilcott (Ireland) Limited, Dundalk Louth (IE)
(72) Inventor: MALCOLM, Karl, Belfast BT5 7HZ Northern Ireland (GB); WOOLFSON, David, Belfast BT9 6TF Northern Ireland (GB)
(74) Representative: Kelly, Donal Morgan
(86) International application number: PCT/EP2004/007703
(87) International publication number: WO 2005/004837

(56) References cited:
- EP-A- 0 577 891
- WO-A-03/020210
- US-A- 1 819 549
- US-A- 3 521 637
- US-A- 4 800 056
- US-A- 4 973 304

## Description

This invention relates to intravaginal drug delivery devices useful in the administration of pharmacologically active agents to a female of the human or animal species.

Although the following description relates primarily to intravaginal rings (IVRs), it is intended that the term intravaginal drug delivery device would embrace all device designs such as, but not limited to, other complete or partial toroid-shaped devices, as well as, ovoid or cylindrical, rectililear or substantially rectlinear, devices.

International Patent Publication No. WO 01/70154 discloses a modified "core" ring design in which there is an open bore extending from the surface into the ring and an active agent-loaded core is then inserted into the open bore, following which the, or each, end of which open bore is then sealed with a cap. Thus, in this modified "core" design, the core is, in use, completely sealed by an outer sheath:
US-A-6,436,428 discloses a further modified "core" ring design, in which there is a bore extending into the ring, from the ring surface and there is a pharmaceutical composition comprising oxybutynin and an excipient, the composition being located in the bore. US-A-6,436,428 suggests that each free end of the bore is subsequently capped and the sealing of both ends of the bore is exemplified in Examples 3, 4, 6 and 8.
WO 99/56934 discloses controlled release devices (defined as at least one rate controlling membrane surrounding a core reservoir), prepared by co-injection moulding. Page 13 teaches that there may be small areas of exposed reservoir material at the entrance gate and/or the exit runner but, by controlling the injection parameters, "exposure of the reservoir material can be eliminated". WO99/56934, in concerning itself with controlled release devices, teaches away from considering providing an incomplete sheath, partly surrounding a core reservoir.
US-A-5,694,947 discloses a non-drug containing core member in the form of an open ring; and a drug containing delivery means, which encircles the core member, along part of its length, in a belt wise manner. The inner surface of the delivery means is in contact with a material which prevents migration of active agent into the core member. The delivery means may be surrounded with a membrane coating whose thickness may be adjusted.
US Patent No. 4,973,304 discloses a sustained release device arranged whereby active substance in a cavity is released through a hydrogel layer covering a port in a wall made of water-impermeable material. US Patent No. 4,800,056 discloses a dispenser comprising a semi-permeable wall with a passageway through which drug is released, the semi-permeable wall surrounding a thermo-responsive drug formulation and an expandable driving member. U.S. Patent No. 1,819,549 discloses a pessary comprising a drug dispersed in gelatine, the gelatine being adapted to become liquid or to dissolve by heat or moisture. U.S. Patent No. 3,521,637 discloses a tampon that, in the Figure 2 embodiment, includes an end wall having perforations. None of D1-D4 disclose or suggest an intravaginal drug delivery device whereby the at least one pharmalogically active agent is dispersed in a hydrophobic elastomeric polymer.

Various physicochemical parameters control the rate of release of a pharmacologically active agent (drug) from any such intravaginal drug delivery device having an outer, rate-controlling sheath (see Chien [Novel Drug Delivery Systems, 2nd Edition, Chapter 2, pp 43-137 (Marcel Dekker)] which is incorporated herein in its entirety).

Problems arise in relation to relatively hydrophilic drugs which may not possess sufficient solubility in the sheath of the intravaginal drug delivery device, and/or whose molecular size/volume/weight are too large for rapid diffusion, to permit sufficient drug delivery to the device's surface and subsequent release.

Generally, drugs with a molecular weight greater than 400 Daltons fall into this latter category. The difficulties are even more considerable when a daily release rate of the drug in the order of milligrams per day, is required.

Accordingly, to overcome these problems a new intravaginal drug delivery device is needed that allows relatively hydrophilic and/or relatively large molecular size/volume/weight drugs to be released from the device at suitable rates.

In the present invention, this is achieved through the use of intravaginal drug delivery devices in which at least part, but not all, of the reservoir is directly exposed to, in use, the vaginal environment. This results in shorter diffusional pathways for drug permeation compared with conventional sheath-enclosed intravaginal drug delivery devices, where the drug must also diffuse through the sheath. Since sheaths are conventionally hydrophobic, this partial "by-passing" of sheath drug permeation permits a wider choice of reservoir carrier materials such as, for example, less hydrophobic carrier systems which, in turn, permits a wider choice of pharmacologically active agent(s).

Accordingly, the invention provides, in a first aspect, an intravaginal drug delivery device, comprising at least one reservoir, the, or each, reservoir containing at least one pharmacologically active agent or a prodrug thereof, dispersed in a carrier system; and a sheath, optionally an elastomeric sheath, discontinuously surrounding the reservoir. Said device may be of any dimensions compatible with intravaginal administration to the human or animal female.

The sheath must discontinuously surround the reservoir in order that part, but not all, of the at least one reservoir is directly exposed, in use, to the vaginal environment. This can be achieved by the provision of one or more, optionally at least two, further optionally at least three or at least five, holes or openings, as will be described in greater detail hereunder. Alternatively, this can be achieved by filling the said holes or openings with further reservoir carrier material, which further reservoir carrier material can be the same or different.

The discontinuous sheath, where present, may be either of substantially constant thickness or its thickness may vary, as is desired.

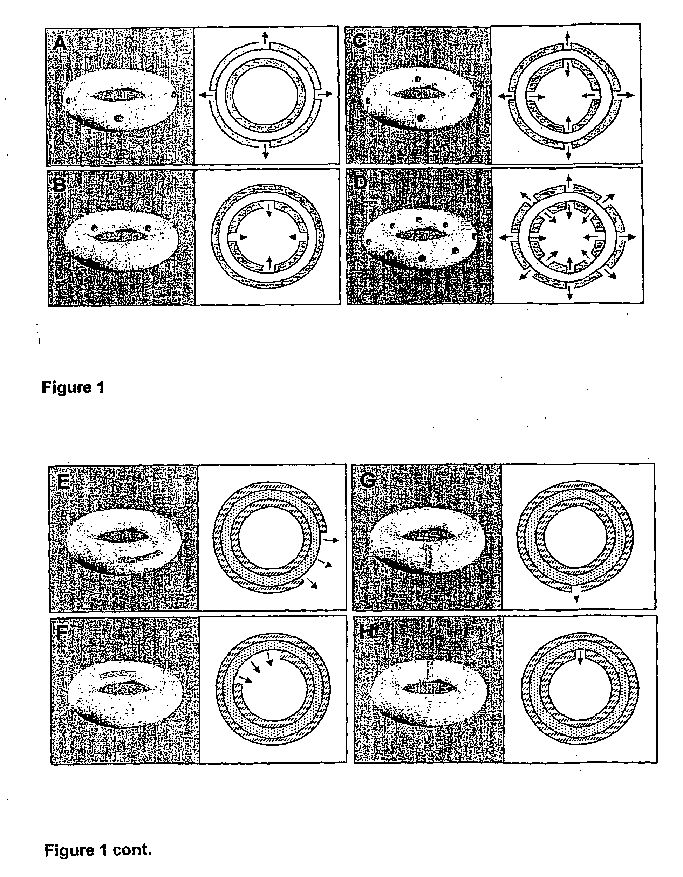

Preferably, the sheath defines one or more, optionally two or more, further optionally at least three or more, holes extending through the sheath to the at least one reservoir, so that part of that reservoir is exposed, in use, to the vaginal environment (Figures 1A to H). These holes may extend to the surface of the at least one reservoir or may, in addition, extend at least partially into the at least one reservoir. These holes may be discrete holes of any shape or may be joined to give a continuous opening in the form of, for example, a slit. Such a slit may extend about the minor circumference of the torus-shaped ring, as shown in Figures 1 G or H or, alternatively, may extend about any major circumference of the torus-shaped ring, as shown in Figures 1 E or F, or, indeed, in any other orientation. The slit may be of any length up to the maximum inner or outer major or minor circumference of a ring device, depending on the location of the slit. Where discrete holes are present in the sheath, they may be present in any size, shape, number, alignment or distribution compatible with the daily rate of drug release required from the device and maintenance of the essential mechanical properties of the device. For example, the total surface area of reservoir exposed to the vaginal environment, *in vivo,* can be in the range of 1-750mm², optionally 5-500mm² or 75-200mm². For rods with a complete sheath over the curvilinear surface and each base partly or completely exposed , a suitable surface area directly exposed to the vaginal environment would be 1-350 mm², for example, 2-150 mm² and, for a ring device or a rod device with holes or openings in the sheath over the curvilinear surface and each base partly or completely exposed, a suitable surface area directly exposed to the vaginal environment could be 25-750 mm², optionally 40-475 or 45 - 250 mm².

For a ring device, said holes or openings are optionally present on the inner circumference of the intravaginal drug delivery device (Figures 1 B, F and H).

Preferably, the direction of said holes or openings are substantially normally arranged relative to the surface of the sheath of the rod or ring device and /or said holes or openings are substantially cylindrical with a diameter in the range of about 0.5 to 6.5 mm, preferably about 1 to 5 mm.

More preferably, there are a plurality, for example 30 or less, optionally20 or less, or further optionally, 2 or 3 to 10 of said holes or slits aligned, optionally linearly, along the surface of the sheath. For a ring device, an arrangement of holes aligned along theinner circumference of the intravaginal drug delivery device (Figures 1 B, F and H) is optional.

The, or each, hole or opening optionally is not in rectilinear or curvilinear alignment with the longitudinal axis of that reservoir. The, or each, hole or opening optionally is not substantially parallel with the longitudinal axis of that reservoir. For example, the, or each, hole or opening may extend at an angle of about 10° to 170°, preferably about 20° to 160°, to the reservoir surface. In a device having a plurality of holes, the angle of each hole may be the same or different.

More particularly, the, or each, hole or opening may extend through the sheath at an angle of 45 to 135°, optionally 70 to 110°, preferably substantially normal to the reservoir surface, but the orientation of the, or each, hole is not intended to be so limited. If the device is a ring device, the, or each, hole may extend substantially radially, inwardly or outwardly, through the sheath.

The device of the present invention may be a partial or complete toroid shape, preferably a partial or complete torus shape or a substantially cylindrical rod. Alternatively, the device of the present invention may be a rod.

Optionally, the sheath may also contain a pharmacologically active agent or a mixture thereof.

The invention is schematically illustrated with reference to the accompanying drawings, in which:
Figure 1 shows perspective and transverse sectional views of various embodiments of the intravaginal drug delivery devices of the present invention, in which Figures 1A and 1B show 4 holes on either the outer or inner circumference, Figures 1C and 1D show either 4 or 8 holes on both of the inner and outer circumferences, Figures 1E and 1F show a longitudinally arranged slit on either the outer or inner circumference and Figures 1 G and 1H show a transversely arranged slit in the outer or inner circumference;
Figure 2 shows the cumulative release of metronidazole from an intravaginal drug delivery devices having 0, 4 or 8 external holes and each containing 10% (w/w) metronidazole, depicted by stars, filled squares or open squares, respectively;
Figure 3 shows the cumulative release of metronidazole from an intravaginal drug delivery device having 0 or 8 external holes and containing 10% (w/w) metronidazole, depicted by open circles and open squares, respectively, and shows the cumulative release of metronidazole from an intravaginal drug delivery device having 0 or 8 extenal holes and containing 20% (w/w) metronidazole, depicted by stars and filled squares, respectively;
Figure 4 shows the cumulative release of metronidazole from an intravaginal drug delivery device having 8 external holes and containing 10% (w/w) metronidazole with, or without, the presence of 30% (w/w) hydroxyethylcellulose in the core, depicted by filled squares and open squares, respectively, as well as devices having 10%(w/w) metronidazole, 30% (w/w) hydroxyethyl cellulose and no holes, depicted by stars; and
Figure 5 shows the cumulative release of metronidazole from an intravaginal drug delivery device having 0 or 8 holes (internal or external) and containing 5% (w/w) metronidazole, depicted by stars, filled squares and open squares, respectively.
Figure 6 shows a perspective diagrammatic view (not to scale) of an intravaginal drug delivery device in the form of a rod having a sheath (clear) partially surrounding a reservoir (shaded) and showing the absence of sheath at each base.
Fig. 7 shows *in vitro* cumulative release of fluoxetine hydrochloride from: □ conventional reservoir ring and ■ perforated ring with 8 external holes.
Fig. 8 shows *in vitro* cumulative release of terconazole from: conventional reservoir ring (TER A) and perforated ring with 8 external holes (TER B).
Fig. 9 shows *in vitro* cumulative release of bovine serum albumin from: □ conventional reservoir ring and perforated ring with 8 external holes (open diamonds).
Fig. 10 shows *in vitro* cumulative release of dextran sulphate from: conventional reservoir ring (open squares) and perforated ring with 8 external holes (closed diamonds).
Fig. 11 shows *in vitro* cumulative release of leuprolide acetate from: conventional reservoir ring (●), perforated ring with 8 external holes (■), slitted ring (□).
Fig. 12 shows *in vitro* cumulative release of desmopressin acetate from: □ conventional reservoir ring and ■ perforated ring with 8 external holes.
Fig. 13 shows *in vitro* cumulative release of clomiphene citrate from: □ conventional reservoir ring and perforated ring with 8 external holes (open triangles).
Fig. 14 shows *in vitro* cumulative release of raloxifene HCl from: □ conventional reservoir ring and perforated ring with 8 external holes (open diamonds).
Fig. 15 shows *in vitro* cumulative release of sumatriptan succinate from: □ conventional reservoir ring and perforated ring with 8 external holes (open triangles).
Fig. 16 shows *in vitro* cumulative release of tamoxifen citrate from: □ conventional reservoir ring and perforated ring with 8 external holes (open triangles).
Fig. 17 shows a representation of a gel filled perforated ring (left) and a gel-filled non-perforated ring (right).
Fig. 18 shows *in vitro* cumulative release of fluxoxetine HCl from: □ conventional reservoir ring and perforated ring with 8 external holes (closed diamonds).
Fig. 19 shows *in vitro* cumulative release of acyclovir from rod-type devices containing 0,10%, 20% or 30% CCM, depicted by open diamonds, open squares, open triangles and stars, respectively.
Fig. 20 shows *in vitro* cumulative release of leuprolide acetate from rod-type devices containing 0,10%, 20% or 30% CCM, depicted by open diamonds, open squares, open triangles and stars, respectively.

In a second aspect of the invention, there is provided a method of manufacturing an intravaginal drug delivery device according to the first aspect of the present invention, said method comprising the steps of combining at least one pharmacologically active agent, and at least one pharmaceutically acceptable carrier system, curing the whole and applying a sheath to discontinuously surround the reservoir.

In a third aspect of the invention, there is provided a method of manufacturing an intravaginal drug delivery device according to the first aspect of the present invention, said method comprising injecting or extruding a reservoir material into a hollow sheath. The sheath may be prior-provided as a discontinuous sheath or, alternatively, the sheath may be subsequently modified to form said discontinuous sheath.

### Intravaginal Devices

Although the following description relates primarily to intravaginal rings (IVRs), it is intended that the term intravaginal drug delivery device would embrace all device designs such as, but not limited to, ovoid or cylindrical devices.

Jackanicz [Jackanicz, T. M., Vaginal Contraception: New Developments. Harper and Row, Hagerstown, pp. 201-212, 1979)] teaches that several designs of intravaginal ring are possible for drug delivery in the vagina.

One ring device is that described as a "matrix" ring, in which the pharmacologically active agent is homogeneously distributed throughout the ring.

Another ring device is that described as a "shell" device, in which a pharmacologically active agent is dispersed in a reservoir, the reservoir being in the form of a narrow band or hollow annulus, sandwiched between a non-medicated central member and an outer non-medicated sheath which wholly surrounds the reservoir. This sheath acts as a metering, or rate-controlling, membrane. With this design, burst effects are reduced, when compared with the "matrix" ring. The "shell" design, with its outer non-medicated sheath, was originally introduced to permit faster release rates than those obtained from conventional "core" devices (see below). However, the disadvantage with the "shell" design is that the drug reservoir volume is limited, because of the non-medicated central member and the non-medicated outer sheath, so that sustained release over long periods is not possible due to drug exhaustion.

Another ring device is that described as a 'core' device in which the pharmacologically active agent is dispersed within a carrier system to form the reservoir, the reservoir being fully surrounded by a sheath designed to control the rate of release of the pharmacologically active agent from the device. In this design, high drug loadings are possible such that prolonged drug release can be achieved for up to twelve months from a single device. Burst release of drug is reduced, as compared to the aforementioned "matrix" ring design. Substantially zero-order release can be achieved due to the presence of the rate-controlling sheath. All commercially available intravaginal ring drug delivery devices are of "core" design and comprise a drug-loaded reservoir, wholly surrounded by a rate-controlling sheath.

Various physicochemical parameters control the rate of release of a pharmacologically active agent (drug) from any ring or rod intravaginal drug delivery device having an outer, rate-controlling sheath (see Chien [Novel Drug Delivery Systems, 2nd Edition, Chapter 2, pp 43-137 (Marcel Dekker)] which is incorporated herein in its entirety).

For the purposes of drug delivery from conventional intravaginal drug delivery devices, which are fully surrounded by rate-controlling sheaths, drugs are usually incorporated into the reservoir at sufficiently high concentrations such that most of the drug is present in the solid state. Before release can occur, individual molecules of the dispersed active drug(s) within the reservoir must first detach themselves from their crystal lattice, dissolve into the surrounding reservoir carrier system, diffuse to the surface of the reservoir and then diffuse through the sheath to the surface of the device. Once at the surface, the drug should then exhibit some aqueous solubility in order to partition into the aqueous diffusion layer consisting primarily of vaginal fluid, from which it then partitions into and across vaginal epithelium and, hence, into the systemic circulation.

The ability of the sheath to be rate-controlling is a function of the solubility and diffusivity of the drug within the sheath. The solubility of the drug in the sheath is determined by its chemical structure/functionality, while the diffusivity of the drug through the sheath is related to its molecular size/volume/weight. Thus, drug solubility in the sheath and relatively small molecular size are thought to be important for significant delivery of a drug to the surface of such a device.

Unfortunately, the sheaths currently employed in the manufacture of intravaginal drug delivery devices are highly hydrophobic in nature [Polymeric Biomaterials: 2nd Edition, (Marcel Dekker) ed. Severian Dumitriu, pp 79-80 (silicone), p332 (poly(ethylene-co-vinyl acetate) and p328 (styrene/butadiene block copolymers)] and are thus best suited, when fabricated as the sheath of an intravaginal drug delivery device, for the intravaginal delivery of hydrophobic active agents such as steroids [AD Woolfson et al. Journal of Controlled Release, 61 (1999) 319-328; LGJ de Leede et al. Contraception 34 (1986) 589-602; SA Ballagh et al. Contraception 50 (1994) 517-533].

Where the device is a pessary, the reservoir takes the form of a core of suitable shape for internalisation within the pessary sheath. Where the device is a "shell" ring device, the, or each, reservoir takes the form of a narrow band or hollow partial or full annulus. Where the device is a "core" ring device, the, or each, reservoir takes the form of a partial or full annulus. Optionally, the partial or full annular reservoir is coaxial, or concentric, with the "shell" or "core" ring device.

In order to extend the concept of the perforated vaginal ring to alternative non-torus vaginal drug delivery devices, a rod-type device is now described which provides release of substances to the human vagina over at least 1-3 days. Such rods may be advantageous in that the reservoir can be small in volume and so can accommodate a high drug loading without undue wastage of the or each pharmacologically active agent, which pharmacologically active agent can be expensive.

Rod-type delivery devices consist of an elastomer sheath partly surrounding a medicated elastomer or semi-solid reservoir. The rod comprises two opposed substantially planar bases linked by a curvilinear surface. The partial sheath can be achieved by either exposing part or all of one or each base (teminal end) of the rod to the vaginal environment and/or providing holes or openings in the curvilinear surface of the sheath.

The second alternative requires the presence of holes or openings in the sheath over the curvilinear surface but not in the sheath over each base so that the sheath over the curvilinear surface is interrupted with one or more, optionally two or more, further optionally, three or more perforations. Preferably, the sheath over the curvilinear surface defines one or more, optionally two or more, further optionally at least three or more, further said holes extending through the sheath to the at least one reservoir, so that part of that reservoir is exposed, in use, to the vaginal environment. These further holes may extend to the surface of the at least one reservoir or may, in addition, extend at least partially into the at least one reservoir. These further holes may be discrete holes of any shape or may be joined to give a continuous opening in the form of, for example, a slit may extend about any major circumference of the rod or in any other orientation. The slit may be of any length up to the maximum inner or outer major or minor circumference of the rod device, depending on the location of the slit. Where discrete holes are present in the sheath, they may be present in any size, shape, number, alignment or distribution compatible with the daily rate of drug release required from the rod device and maintenance of the essential mechanical properties of the rod device. For rod devices, a suitable surface area directly exposed to the vaginal environment would be 1 - 450 mm², for example, 2-75 mm² or 2-50mm².

Preferably, the direction of said holes or openings are substantially normally arranged relative to the surface of the sheath of the rod device and /or said holes or openings are substantially cylindrical with a diameter in the range of about 0.5 to 6.5 mm, preferably about 1 to 5 mm.

More preferably, there are a plurality, for example 30 or less, optionally20 or less, or further optionally, 2 or 3 to 10 of said holes or slits aligned, optionally linearly, along the surface of the sheath.

The, or each, hole or opening optionally is not in rectilinear or curvilinear alignment with the longitudinal axis of that reservoir. The, or each, hole or opening optionally is not substantially parallel with the longitudinal axis of that reservoir. For example, the, or each, hole or opening may extend at an angle of about 10° to 170°, preferably about 20° to 160°, to the reservoir surface. In a device having a plurality of holes, the angle of each hole may be the same or different.

More particularly, the, or each, hole or opening may extend through the sheath at an angle of 45 to 135°, optionally 70 to 110°, preferably substantially normal to the reservoir surface, but the orientation of the, or each, hole is not intended to be so limited.

In the first alternative, the sheath completely surrounds the curvilinear surface and the partial or complete exposure of the, or each, base is achieved by partial or complete absence of sheath over the, or each, base. In one optional embodiment, the sheath surrounds the curvilinear surface but is absent at each base and, in this embodiment, the hole substantially corresponds to the dimensions of each end of the reservoir. In this optional embodiment, the distance between the opposed bases (length of the rod device) may be 1- 50mm, optionally 1-30 mm, since active is primarily released in this optional embodiment through the or each partially or fully exposed base.

In the third alternative, where part or all of at least one base is exposed and, in addition, holes or openings are provided in the sheath, in which event, the or each opposing base of the rod-type delivery device is directly exposed, in use, to the vaginal environment and the sheath is interrupted with one or more, optionally two or more, further optionally, three or more perforations. Preferably, the sheath defines one or more, optionally two or more, further optionally at least three or more, further said holes extending through the sheath to the at least one reservoir, so that part of that reservoir is exposed, in use, to the vaginal environment. These further holes may extend to the surface of the at least one reservoir or may, in addition, extend at least partially into the at least one reservoir. These further holes may be discrete holes of any shape or may be joined to give a continuous opening in the form of, for example, a slit may extend about any major circumference of the rod or in any other orientation. The slit may be of any length up to the maximum inner or outer major or minor circumference of the rod device, depending on the location of the slit. Where discrete holes are present in the sheath, they may be present in any size, shape, number, alignment or distribution compatible with the daily rate of drug release required from the rod device and maintenance of the essential mechanical properties of the rod device. For rod devices, a suitable surface area directly exposed to the vaginal environment would be 1- 450 mm², for example, 2-75 mm².

Preferably, the direction of said holes or openings are substantially normally arranged relative to the surface of the sheath of the rod device and /or said holes or openings are substantially cylindrical with a diameter in the range of about 0.5 to 6.5 mm, preferably about 1 to 5 mm.

More preferably, there are a plurality, for example 30 or less, optionally20 or less, or further optionally, 2 or 3 to 10 of said holes or slits aligned, optionally linearly, along the surface of the sheath.

The, or each, hole or opening optionally is not in rectilinear or curvilinear alignment with the longitudinal axis of that reservoir. The, or each, hole or opening optionally is not substantially parallel with the longitudinal axis of that reservoir. For example, the, or each, hole or opening may extend at an angle of about 10° to 170°, preferably about 20° to 160°, to the reservoir surface. In a device having a plurality of holes, the angle of each hole may be the same or different.

More particularly, the, or each, hole or opening may extend through the sheath at an angle of 45 to 135°, optionally 70 to 110°, preferably substantially normal to the reservoir surface, but the orientation of the, or each, hole is not intended to be so limited.

The reservoir contains the therapeutic agent (0.001 % to 80% w/w, , optionally 0.005% to 65% w/w, preferably 0.005% w/w to 30%w/w) and optionally a release-modifying substance (1% to 80% w/w, optionally 5% to 50% w/w) for the purposes of modifying the release characteristics of the therapeutic agent. Examples of non-therapeutic agents include, but are not limited to, polyethylene glycerol, glucose, glycine, ascorbic acid, hydroxyethylcellulose, croscarmellose, lactose but reference is made to the teaching elsewhere herein of suitable excipients.

If the rod-type device has no sheath at each end (or base), release of large molecular weight and/or hydrophilic therapeutic agent occurs predominantly from the open bases of the rod-type devices, while small hydrophobic compounds may also be released via permeation through the sheath layer. One purpose of the release-modifying agent contained within the reservoir of the device is to absorb water and thus enhance the release of the therapeutic agent.

Rod-type silicone devices containing a medicated silicone matrix reservoir may be manufactured by (i) coextrusion of (A) non-medicated sheath elastomer and (B) medicated reservoir elastomer through a concentrically arranged die, or (ii) injection of the medicated silicone reservoir elastomer mix into a pre-formed non-medicated sheath silicone tube.

Rod-type silicone devices containing a medicated semi-solid resevoir may be manufactured by injection of a medicated semi-solid formulation into a preformed silicone sheath tube.

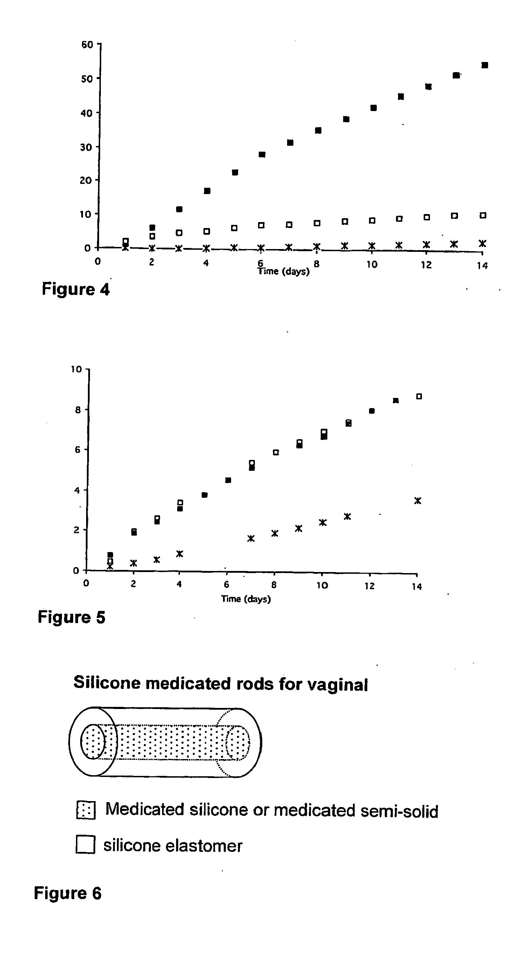

The rod-type devices may be of any shape capable of being accommodated in the human vagina, although substantially cylindrical is preferred. By cylinder is meant a body bounded by two parallel plane bases and a curved surface generated by moving along a fixed curve while staying parallel to its original position. One such cylinder is a right cylinder, whose bases are normal to the generatrix - such a right cylinder is illustrated in Figure 6 of the accompanying drawings, although the invention is not intended to be limited to right cylinders. The terminal edges of the terminal ends (or bases) of the rods may also be rounded or chamfered to eliminate sharp edges.

The dimensions of the rod-type silicone delivery devices should be such that they are capable of being administered and retained within the anatomical constraints of human vagina. For this purpose, rod-type devices may range from 1 to 30mm, optionally 2 to 10 mm, cross-sectional diameter, and 2 to 80mm, optionally 5 to 40 mm, in length. The rod-type devices may also optionally contain a string attached to aid removal from the vagina.

### Reservoir

The reservoir carrier system should be, in use, solid or semi-solid, i.e. capable of conforming to the shape of the space available for the reservoir, e.g., fabricated from a material selected from a shape retaining material; a thermosetting material; or a thermoplastic material. The reservoir carrier system comprises an elastomeric hydrophobic polymer and must be biocompatible, i.e., suitable for insertion in the human or animal body.

The reservoir carrier system is chosen to achieve desirable drug release therefrom.

The dimensions of the reservoir are determined by such factors as the amount of drug to be delivered to the subject; the time period over which the drug is to be delivered; and the permeation characteristics of the drug.

Examples of suitable polymeric reservoir materials include silicones, poly(ethylene-co-vinyl acetate), and styrene-butadiene-styrene block copolymers.

Elastomers are defined as amorphous, or predominantly amorphous, high molecular weight polymers above their glass transition temperature, which can be stretched and retracted rapidly, exhibit high strength and modulus when stretched, and recover fully whenever the stress is removed. Generally, these elastomers are crosslinked to restrain gross mobility, either permanently (a covalently-crosslinked elastomer), or reversibly (a thermoplastic elastomer). Elastomers are typically chosen from the room-temperature vulcanising type of organopolysiloxanes, for example, poly(dimethylsiloxane). Non-silicone elastomers that are known in the art include poly(ethylene-co-vinyl acetate) [Roumen FJME, Dieben TOM, Contraception, 59 (1999) 59-62] and styrene-butadiene-styrene block copolymer [Vartiainen J, Wahlstrom T, Nilsson CG, Maturitas, 17 (1993) 129-137].

A preferred reservoir carrier system is derived from hydroxyl-terminated organopolysiloxanes (such as those disclosed in US-A-5,855,906) of the RTV (room temperature vulcanising) type, which harden to elastomers at room temperature or higher, following the addition of cross-linking agents in the presence of curing catalysts. The ability to crosslink at room temperature is, of course, desirable for the delivery of thermally sensitive pharmacologically active agents. Suitable cross-linking agents and curing catalysts are well known in the art. Typical curing catalysts would be the organic metal compounds such as stannous octoate, dibutyltin dilaurate, alkyl titanates, platinum systems and titanium chelates. The curing catalyst is chosen so as to be effective in the presence of the drug and not to interact chemically with the drug. Typical crosslinking agents would be alkoxysilanes such as tetraethoxysilane or n-propylorthosilicate (NPOS). Curing temperatures and times will vary, depending on the particular elastomer(s) used. For example, the curing temperature may vary between room temperature (15-25°C) and 150°C but is preferably within the range 60-90°C. The curing time may vary between a few seconds and several hours, depending on the elastomer(s) used. A preferred reservoir material is commercially available as Nusil Med 7.6382 from Nusil Technology, Carpinteria, California, USA.

Other suitable silicone elastomers suitable for intravaginal ring reservoir manufacture include addition-type, two-component poly(dimethylsiloxane)s which are platinum catalysed at room temperature or under elevated temperatures, one-component poly(dimethylsiloxane)s, and silicone elastomers functionalised with fluorine, benzyl and other moieties.

The reservoir, irrespective of its carrier material, may optionally contain 1 to 80%w/w, optionally 5-50%w/w of one or more pharmaceutically acceptable excipients designed to further enhance the rate of drug release from the device. Examples include, but are not limited to water-soluble or water-swellable polysaccharides, preferably cellulose derivatives such as croscarmellose (crosslinked carboxymethylcellulose) or hydroxyethylcellulose, glucose, lactose or other mono- or di-saccharides,or their water-soluble salts, proteins such as gelatin, nonionic surface active agents, bile salts, organic solvents, such as ethoxydiglycol, polyethylene glycol and fatty acid esters, preferably containing 2 to 20 carbon atoms, of which myristate esters are preferred.

Pharmaceutically acceptable fillers may be added to enhance the mechanical strength of the reservoir. For example, suitable fillers include finely divided, reinforcing or extending fillers such as high surface area fumed and precipitated silicas, clays such as kaolin, crushed quartz, diatomaceous earths, calcium carbonate, barium sulphate, iron oxide, titanium dioxide and carbon black. The proportion of fillers added will depend on the desired properties of the cured device but, usually, the filler content of the reservoir will be in the range 5-35 parts by weight, optionally 7.5-27.5 parts by weight, per 100 parts by weight of the aforementioned reservoir carrier system.

Where the device is an intravaginal drug delivery device in the form of a ring, the reservoir may be a full reservoir, in that it forms a continuous (or annular) reservoir within the device, or it may be a partial reservoir, in that the reservoir is of a defined length, which is discontinuous. Optionally, more than one partial reservoir may be used in the same device, where each reservoir may contain the same pharmacologically active agent, different pharmacologically active agents, and/or more than one agent. Where one or more partial reservoirs are used, at least one, but preferably each, reservoir must be partially exposed, in use, to the vaginal environment via, for example, at least one hole extending from the surface of the sheath through to at least the surface of the at least one, but preferably each, reservoir.

It will be appreciated that at least some of the drug is released from the reservoir by diffusion of the drug through the reservoir carrier system. Among the important factors governing release from the intravaginal drug delivery devices of the present invention are the solubility of the drug in the reservoir carrier system, the solubility of the reservoir carrier material and/or reservoir excipient in vaginal fluid, the surface area of the reservoir exposed to the vaginal environment and the distance the drug must diffuse within the reservoir carrier system to reach this "exposed" surface area.

### Sheath

The sheath, which discontinuously surrounds the reservoir, comprises polymer which is biocompatible, i.e., suitable for insertion in the human or animal body. The sheath may, or may not, be capable of permitting the, or each, agent to diffuse therethrough. Polymeric and non-polymeric materials, as used in the aforementioned core, are also suitable for use in the sheath, whether or not they are elastomeric. For example, poly(ethylene-co-vinylacetate), styrene-butadiene block copolymers, polyurethanes, and silicones are mentioned, of which silicones are preferred. However, silicone elastomers need not be functionalised with fluorine.

More preferably, the polymer is an elastomer, particularly if the reservoir carrier system is not elastomeric. In this embodiment, the elastomeric properties of the sheath confer sufficient flexibility on the composite intravaginal drug delivery device to allow placement in, and retention within, the vagina. Most preferably, the polymer is a silicone elastomer derived from hydroxyl-terminated organopolysiloxanes (such as polydimethylsiloxanes) of the RTV type, which cure to elastomers at room temperature or higher, following the addition of cross-linking agents in the presence of curing catalysts.

Other suitable silicone elastomers suitable for intravaginal ring sheath manufacture include addition-type, two-component poly(dimethylsiloxane)s which are platinum catalysed at room temperature or under elevated temperatures, one-component poly(dimethylsiloxane)s, and silicone elastomers functionalised with benzyl and other moieties.

Preferably, the sheath may also contain fillers to enhance the mechanical strength of the sheath. Fillers suitable for use in the reservoir are also suitable for use in the sheath. Usually, the filler content of the sheath will be in the range 0 to 35 parts by weight per 100 parts by weight of the sheath carrier system.

The sheath may also optionally contain one or more additional pharmacologically active agents.

The sheath may also optionally contain at least one pharmaceutically acceptable excipient designed to reduce or prevent drug release from the reservoir via diffusion through the sheath. Such excipients are often the same materials used as fillers, and act so as to increase the tortuosity of the diffusional path of the active agent, i.e., increase the diffusional distance that the active agent must traverse through the device prior to its release from said device. For example, suitable diffusion inhibitors include high surface area fumed and precipitated silicas, clays such as kaolin, crushed quartz, diatomaceous earths, calcium carbonate, barium sulphate, iron oxide, titanium dioxide and carbon black.

The sheath may further optionally contain at least one pharmaceutically acceptable chemical penetration enhancers designed to enhance drug absorption across the vaginal epithelium, for example, surface active agents, agents that have a reversible effect on the arrangement of epithelial lipids, such as oleic acid or agents that directly affect tight junctions between epithelial cells.

### Device Geometry

The geometry of the device of the present invention may be chosen according to theoretical calculations by methods known to those skilled in the art such that the desired daily release of the at least one pharmacologically active agent is achieved and sustained for the desired duration of, for example, 1-14 days, optionally, 3-7 days. For example, in the examples which follow, we have demonstrated that, over 5 days, it is possible to release a total of 0.001-250mg, optionally 0.01-150mg, into a disssolution fluid chosen to represent "sink conditions" (as defined hereinafter) under the exemplified release conditions.

For an intravaginal drug delivery device, the desired "geometry" would encompass, for example, the length, width and cross-sectional area of the device. For an intravaginal ring, the term "geometry" encompasses the overall diameter of the ring, the cross-sectional diameter of the ring and the length of the reservoir. Where the intravaginal ring is of "core" design, the term "geometry" also includes the ratio of the reservoir diameter to the diameter of the complete device in cross-section. A preferred geometry is a ring of "core" design having an overall or outer diameter of 45 - 60 mm, preferably 52-58mm; a reservoir diameter 1-7 mm, optionally 2-6.5mm, preferably 3-6mm; a cross-sectional diameter of 4 - 10 mm, optionally 4.5 - 10 mm, preferably 6.5-9.5mm; and a reservoir length of 2 - 200 mm. Another preferred geometry is a substantially cylindrical rod having an overall or outer diameter of 1 - 30 mm, preferably 2-10mm; a reservoir diameter 0.5 - 6 mm, optionally 1.5-6mm, preferably 2.5-5mm; and an overall length of 1 - 80mm, optionally 5 - 40mm.

### Pharmacologically Active Agents

By "pharmacologically active agent" is meant any agent capable of defending against, or treating, a disease state in the human or animal body, or a prodrug thereof. Such agents are intended to be released into vaginal fluid by diffusion out of the intravaginal drug delivery device, and may exert their effect either locally or systemically. The active agent(s) may be hydrophilic or lipophilic, organic or inorganic material(s), which are prophylactically or therapeutically active.

By "prophylactic agent" is meant any agent (or its prodrug) effective in defending against a disease state in the human or animal body, preferably the human body.

By "therapeutic agent" is meant any agent (or its prodrug) effective in treating a disease state in the human or animal body, preferably the human body.

The terms "agent", "active agent" and "drug" are used herein interchangeably and are intended to mean any substance which falls within the definition of a prophylactic agent or a therapeutic agent and which is capable *in vivo* of producing a desired, usually beneficial, effect.

Suitable prophylactic or therapeutic agents for use in reservoirs and/or sheaths in the devices of the present invention include, but are not limited to, the following:
- **Contraceptive drugs**
   Desogestrel, Dienestrol, Diethylstilberol, Estradiol, Estriol, Estradiol-3-acetate, Ethinyl Estradiol, Etonogestrel, Gestodene, Levonorgestrel, Medroxyprogesterone, Medroxyprogesterone Acetate, Mestranol, Norethisterone, Norgestimate, Nonoxynol-9, Norethisterone Acetate, Progesterone, Testosterone, Testosterone Acetate, ST-1435 (a progestin), Tibolone
- **Pain and Migraine**
   5HT-1 receptor blockers such as Almotriptan, Eletriptan, Frovatriptan, Naratriptan, Rizatriptan, Sumatriptan, Zolmatriptan
- **Drugs for hormone replacement therapy**
   Dehydroepiandrosterone sulphate, Dienestrol, Diethylstilberol, Estrogens such as Estradiol, Estriol, Estradiol-3-acetate, Ethinyl Estradiol, Gestodene, Levonorgestrel, Luteinizing Hormone Releasing Hormone, Norethisterone, Norethisterone Acetate, Progesterone, ST-1435, Testosterone, Testosterone Acetate
- **Anxiety and Depression**
   Selective Serotonin Reuptake Inhibitors (SSRIs) such as fluoxetine
- **PreMenstrual Syndrome**
   Selective Serotonin Reuptake Inhibitors (SSRIs) such as fluoxetine
- **Drugs for genito-urinary disorders**
   Flavoxate Hydrochloride, Propantheline Bromide, Tolterodine Tartrate
- **Drugs for cervical ripening / induction of labour**
   misoprostol, oxytocin, PGE2, dinoprostone, nitric oxide donors(i.e., isosorbide mononitrate)
- **Antibacterial drugs**
   Acrosoxacin, Amifloxacin, Amoxycillin, Ampicillin, Aspoxicillin, Azidocillin, Azithromycin, Aztreonam, Balofloxacin, Benzylpenicillin, Biapenem, Brodimoprim, Cefaclor, Cefadroxil, Cefatrizine, Cefcapene, Cefdinir, Cefetamet, Cefmetazole, Cefprozil, Cefroxadine, Ceftibuten, Cefuroxime, Cephalexin, Cephalonium, Cephaloridine, Cephamandole, Cephazolin, Cephradine, Chlorquinaldol, Chlortetracycline, Ciclacillin, Cinoxacin, Ciprofloxacin, Clarithyromycin, Clavulanic Acid, Clindamycin, Clofazimine, Cloxacillin, Danofloxacin, Dapsone, Demeclocycline, Dicloxacillin, Difloxacin, Doxycycline, Enoxacin, Enrofloxacin, Erythromycin, Fleroxacin, Flomoxef, Flucloxacillin, Flumequine, Fosfomycin, Isoniazid, Levofloxacin, Mandelic Acid, Mecillinam, Metronidazole, Minocycline, Mupirocin, Nadifloxacin, Nalidixic Acid, Nifurtoinol, Nitrofurantoin, Nitroxoline, Norfloxacin, Ofloxacin, Oxytetracycline, Panipenem, Pefloxacin, Phenoxymethylpenicillin, Pipemidic Acid, Piromidic Acid, Pivampicillin, Pivmecillinam, Prulifloxacin, Rufloxacin, Sparfloxacin, Sulbactam, Sulfabenzamide, Sulfacytine, Sulfametopyrazine, Sulphacetamide, Sulphadiazine, Sulphadimidine, Sulphamethizole, Sulphamethoxazole, Sulphanilamide, Sulphasomidine, Sulphathiazole, Temafloxacin, Tetracycline, Tetroxoprim, Tinidazole, Tosufloxacin, Trimethoprim and salts or esters thereof.
- **Antifungal drugs**
   Suitable antifungal agents include Bifonazole, Butoconazole, Chlordantoin, Chlorphenesin, Ciclopirox Olamine, Clotrimazole, Eberconazole, Econazole, Fluconazole, Flutrimazole, Isoconazole, Itraconazole, Ketoconazole, Miconazole, Nystatin, Nifuroxime, Terconazole, Tioconazole, Undecenoic Acid and salts or esters thereof.
- **Antimalarial agents**
   Chloroquine and Dapsone.
- **Antiprotozoal agents**
   Acetarsol, Aminacrine, Azanidazole, Metronidazole, Nifuratel, Nimorazole, Ornidazole, Propenidazole, Secnidazole, Sinefungin, Tenonitrozole, Ternidazole, Tinidazole and salts or esters thereof.
- **Antiviral drugs, including antiretroviral agents**
   AMD3100, N-Acetyl Cysteine, Abacavir, Aciclovir, 3'-Azidothymidine, BCH-10618, Brivudine, CD4, CD4-Ig2, CD4-PEG, CCR5 antagonists, C31G, Cantanospermine, Capravirine, Carrageenan, Cellulose Acetate Phthalate, Cidofovir, Curcumin, DAPD, Desciclovir, Dextrin Sulfate, 2',3'-Dideoxyinosine, 2',3'-Dideoxycytidine, Defensins, Didanosine, 1-Docosanol, Edoxudine, Efavirenz, Emivirine, Emtricitabine, Famciclovir, Fiacitabine, Gramicidin, Ibacitabine, Imiquimod, Immunoglobulins, Indinavir, Lamivudine, Loviride, Magainins, Nevirapine, Nonoxynol-9, Penciclovir, PRO 542, PRO 140, Protegrins, Procysteine, Ritonavir, Saquinavir, TMC-120, TMC-125, TMC-126, Tenofovir, UC-781, Valaciclovir, Valganciclovir and salts or esters thereof, Zalcitabine, Zidovudine
- **Drugs for treatment of endometriosis**
   Danazol
- **Peptides for vaginal administration**
   Adrenocorticotropic Hormone, Angiotensin, Beta-endorphin, Bombesin, Calcitonin, Calcitonin Gene Relating Polypeptide, Cholecystokinin-8, Desmopressin, Endothelin, Enkephalin, Gastrins, Glucagon, Human Atrial Natriuretic Polypeptide, Insulin, Luteinising Hormone Release Hormone, Melanocyte Stimulating Hormone, Muramyl-dipeptide, Neurotensin, Oxytocin, Parathyroid Hormone, Peptide T, Secretin, Somatomedins, Somatostatin, Thyroid Stimulating Hormone, Thyrotropin Releasing Hormone, Thyrotropin Stimulating Hormone, Vasoactive Intestinal Polypeptide, Vasopressin, and their analogues or derivatives.
- **Anti-Emetic Drugs**
   5HT3 antagonists, ondansetron,
- **Osteoporosis and/or hormone replacement therapy**
   Selective Estrogen Receptor Modulators (SERMs) such as raloxifene
- **Other potential drugs for vaginal administration**
   Bromocriptine, clomiphene, tamoxifen, leuprolide

Preferably, the, or each, drug is present in the rod or ring reservoir in an amount of 0.005% to 65% (w/w), optionally 0.5% to 50%(w/w), of the reservoir. Optionally, the, or each, drug is present in the sheath in an amount of 0.001 % to 65% (w/w) of the sheath.

Intravaginal drug delivery devices of the present invention may be prepared by injecting or extruding a reservoir material into a hollow sheath. The sheath may be prior-provided with one or more holes or openings. Alternatively, said one or more holes or openings may subsequently be formed.

Intravaginal drug delivery devices of the present invention may alternatively be prepared by applying a sheath onto a solid reservoir. Once again, the sheath may be prior-provided with one or more holes or openings, or, alternatively, said one or more holes or openings may be subsequently formed.

The intravaginal drug delivery devices of the present invention need not be formed by co-injection of the reservoir material and the sheath.

Embodiments of the invention will now be demonstrated by reference to the following General Method of Manufacture, which are then exemplified by reference to Examples 1 - 17.

The invention is not limited to the embodiments described and exemplified herein, which may be modified and amended without departing from the scope of the present invention. Thus, for instance, it will be obvious to those skilled in the art that the technique of injection moulding referred to herein may be replaced in whole or in part by other manufacturing techniques that will produce the same end product, notably the technique of extrusion.

### General Method of Intravaginal Device Manufacture - Examples 1-4

A hydrophobic elastomeric polymer containing about 25% (w/w) diatomaceous earth as a filler is provided. 97 parts by weight of this polymer is blended with 2.5 parts by weight of a cross-linking agent, n-propylorthosilicate (NPOS), to form an elastomer mix. A suitable hydrophobic elastomeric polymer is stannous octoate-cured polydimethylsiloxane polymer, a suitable example of which is that known as Nusil Med 7.6382.

85 parts by weight of the elastomer mix is further blended with 5 parts by weight of barium sulphate and the required number of parts by weight of the desired pharmacologically active agent(s), to form an active reservoir mix.

The reservoir of the intravaginal drug delivery device of the invention is prepared by mixing 200 parts by weight of the active reservoir mix with 1 part by weight of an activating catalyst, for example, stannous octoate. This mix may, if desired, be placed under vacuum to remove air. The resultant reservoir mix is injected into a reservoir mould and cured at 80°C for 2 minutes. Alternatively, the mix may be extruded, depending on its viscosity. The mould is then opened, following which the reservoir is removed and trimmed. It will be appreciated that, by the use of different reservoir moulds, reservoirs of different lengths or diameters may be produced.

An intravaginal drug delivery device in the form of a complete torus-shaped ring is produced by mixing 200 parts by weight of the elastomer mix with 1 part by weight of an activating catalyst, for example, stannous octoate. The resultant full ring mix is injected into a full ring mould (designed with one or more projections such that corresponding one or more holes extend from the surface of the device at least to the surface of the reservoir will result when the final device is cured) containing the reservoir (full or partial length) prepared as previously described, and then cured at 80°C for 2 minutes. The mould is then opened, following which the full ring is removed and trimmed. A half or part ring could, equally, be prepared by using the required half ring or part ring mould. Furthermore, the full ring might be prepared by placing a pre-assembled half or part ring in the full ring mould and then injecting the full ring mix.

The moulds, which are preferably coated with, for example, Teflon (Trade Mark) or an electrolytically applied metalised coating, may be constructed of hardened carbon steel, stainless steel, aluminium, or any other material deemed to be appropriate. It will be appreciated that the mould design imparts the physical shape of the intravaginal drug delivery device, for example, a partial or complete ring, a rod or any other desired shape. Preferably, the device has a partial or complete toroidal shape, more preferably a partial or complete torus shape, or a substantially cylindrical shape. By toroid is meant a ring-like body generated by rotating any closed loop (including an ellipse, a circle or any irregegular curve) about a fixed line external to that loop. The toroid shape may be a complete or partial toroid. By torus is meant a ring-like body generated by rotating a circle about a fixed line external to the circle. The torus shape may be a complete or partial ring- like shape.

The geometric characteristics of the device and the size, number, distribution, alignment and shape of the holes (openings) can be varied as required by the use of appropriately sized (and angled) inwardly extending projections from the moulds. Alternatively, the intravaginal ring device, or components thereof, may be prepared by extrusional processes, as will be obvious to those skilled in the art. Alternatively, the holes or openings may be introduced into a final ring device by mechanical means, such as a bore device.

### General Method of Intravaginal Device Manufacture - Examples 5-8

A hydrophobic elastomeric polymer (PDMS) containing about 10% (w/w) diatomaceous earth as a filler is provided. 94.24 parts by weight of this polymer is blended with 5.76 parts by weight of a cross-linking agent, n-propylorthosilicate (NPOS), to form an elastomer mix. A suitable hydrophobic elastomeric polymer is stannous octoate-cured polydimethylsiloxane polymer, a suitable example of which is that known as Nusil Med 7.6382.

The elastomer mix is further blended with the desired amount by weight of the desired pharmacologically active agent(s), to form an active reservoir mix.

The reservoir of the intravaginal drug delivery device of the invention is prepared by mixing 200 parts by weight of the active reservoir mix with 1 part by weight of an activating catalyst, for example, stannous octoate. This mix may, if desired, be placed under vacuum to remove air. The resultant reservoir mix is injected into a reservoir mould and cured at 80°C for 3 minutes. Alternatively, the mix may be extruded, depending on its viscosity. The mould is then opened, following which the reservoir is removed and trimmed. It will be appreciated that, by the use of different reservoir moulds, reservoirs of different lengths or diameters may be produced.

An intravaginal drug delivery device in the form of a complete torus-shaped ring is produced by mixing 200 parts by weight of an elastomer mix containing a hydrophobic elastomeric polymer (PDMS) with about 22% (w/w) diatomaceous earth as a filler, with 1 part by weight of an activating catalyst, for example, stannous octoate. The resultant full ring mix is injected into a full ring mould (designed with one or more projections such that corresponding one or more holes extend from the surface of the device at least to the surface of the reservoir will result when the final device is cured) containing the reservoir (full or partial length) prepared as previously described, and then cured at 80°C for 3 minutes. The mould is then opened, following which the full ring is removed and trimmed. A half or part ring or a rod could, equally, be prepared by using the required half ring or part ring or rod mould. Furthermore, the full ring might be prepared by placing a pre-assembled half or part ring in the full ring mould and then injecting the full ring mix.

The rings of Examples 5-8 have a cross-sectional diameter of 7.6mm and an overall diameter of 56mm, with a reservoir, substantially centrally arranged therein, containing 30% by weight of active and having a reservoir diameter of 4.5mm - the sheath has, therefore, a thickness of 1.55mm.

### General Method of Intravaginal Device Manufacture - Examples 9-14

A hydrophobic elastomeric polymer (PDMS) containing about 10% (w/w) diatomaceous earth as a filler is provided. 94.24 parts by weight of this polymer is blended with 5.76 parts by weight of a cross-linking agent, n-propylorthosilicate (NPOS), to form an elastomer mix. A suitable hydrophobic elastomeric polymer is stannous octoate-cured polydimethylsiloxane polymer, a suitable example of which is that known as Nusil Med 7.6382.

The elastomer mix is further blended with 30% by weight of lactose as excipient and with 5% by weight of the desired pharmacologically active agent(s), to form an active reservoir mix.

The reservoir of the intravaginal drug delivery device of the invention is prepared by mixing 200 parts by weight of the active reservoir mix with 1 part by weight of an activating catalyst, for example, stannous octoate. This mix may, if desired, be placed under vacuum to remove air. The resultant reservoir mix is injected into a reservoir mould and cured at 80°C for 3 minutes. Alternatively, the mix may be extruded, depending on its viscosity. The mould is then opened, following which the reservoir is removed and trimmed. It will be appreciated that, by the use of different reservoir moulds, reservoirs of different lengths or diameters may be produced.

An intravaginal drug delivery device in the form of a complete torus-shaped ring is produced by mixing 200 parts by weight of an elastomer mix containing a hydrophobic elastomeric polymer (PDMS) with about 22% (w/w) diatomaceous earth as a filler, with 1 part by weight of an activating catalyst, for example, stannous octoate. The resultant full ring mix is injected into a full ring mould (designed with one or more projections such that corresponding one or more holes extend from the surface of the device at least to the surface of the reservoir will result when the final device is cured) containing the reservoir (full or partial length) prepared as previously described, and then cured at 80°C for 3 minutes. The mould is then opened, following which the full ring is removed and trimmed. A half or part ring or a rod could, equally, be prepared by using the required half ring or part ring or rod mould. Furthermore, the full ring might be prepared by placing a pre-assembled half or part ring in the full ring mould and then injecting the full ring mix.

The rings of Examples 9-14 have a cross-sectional diameter of 7.6mm and an overall diameter of 56mm, with a reservoir, substantially centrally arranged therein, containing 5% by weight of active and having a diameter of 4.5mm, with a sheath thickness of 1.55mm.

### Protocol for In Vitro Release Studies

All of the *in vitro* daily release profiles for the intravaginal ring or rod devices of the invention were determined under sink conditions. The term 'sink conditions' is intended to refer to that set of experimental conditions *in vitro* that effectively simulates the active haemoperfusion that occurs *in vivo,* and which results in a maximum drug diffusion rate, at any given time, across the aqueous boundary layer. Thus, the solubility characteristics of the drug will determine the choice of a suitable dissolution medium.

The *in vitro* daily release profiles for the intravaginal ring devices of Examples 1-4 of the invention were determined under sink conditions in pH 5.0 acetate buffer. The release profiles for Examples 5-8 were again determined under sink conditions - the respective dissolution media were 0.9% saline, 0.9% saline, 0.2% SLS (sodium lauryl sulphate, a surfactant) and pH6.8 water. The release profiles for each of Examples 9-15 were determined under sink conditions in 0.9% saline. For all of Examples 1-14, the volume of dissolution medium was 100ml in each case and this dissolution medium was changed daily, whilst for Example 15, the volume of dissolution medium was 50ml and this dissolution medium was changed daily. For Examples 16 and 17, the volume of dissolution was 10ml of deionised water and, again, this dissolution medium was changed daily.

Release rates were determined in the following manner. Each intravaginal ring (n=at least 2) was suspended in 100ml (or 10ml for Examples 16 and 17) of the dissolution medium chosen to achieve sink conditions for that active in an individual stoppered 250 ml conical flask. The flasks were maintained at a constant temperature of 37°C in a shaking incubator. The contents of each flask were gently shaken at a constant rate (60 rotations per minute) selected to ensure the absence of a hydrostatic layer on the surface of the ring. The 100ml (or 10 ml for Examples 16 and 17) of that dissolution medium was renewed every 24 hours (± 15 minutes) over the desired period. An aliquot (2 ml) of the used dissolution medium was analysed by high-performance liquid chromatography.

In Examples 1-4, the geometry of the rings was as follows: 9 mm (transverse cross-sectional diameter), 54 mm (outer diameter), 5.5 mm (reservoir transverse cross-sectional diameter). The sheath thickness was 1.75 mm, and the cross-sectional diameter of each hole was 3.0 mm with a hole depth of at least 1.75 mm.

In Examples 5-15, the geometry of the rings was as follows: 7.6 mm (transverse cross-sectional diameter), 56 mm (outer diameter), 4.5 mm (reservoir transverse cross-sectional diameter). The sheath thickness was 1.55 mm, and the cross-sectional diameter of each hole was 4.0 mm (a "size 1 core bore") with a hole depth of about 2.5 mm.

### Protocol for Analysis of Release into Dissolution Medium

| **Conditions** | **Acyclovir** | **Fluoxetine HCl** | **Leuprolide acetate** |
|---|---|---|---|
| Column | Hypersil | Zorbax SB-C8 | Nucleosil C18 |
| | BDS C18 250x4.6mm, 5µm | 75x3.5mm, 5µm | 100x4.6mm, 5µm |
| Detection λₙₘ(nm) | 254 | 227 | 215 |
| Inj Vol (µL) | 20.0 | 20.0 | 10.0 |
| Mobile | Acetic acid solution (0.1 % w/w) | Pentane-sulphonic | A; 5mM HCl: |
| Phase | | | MeCN 72:28 |
| | | Acid:MeOH | B; 5mM HCl: |
| | | 33:67 (pH 5) | MeCN 65:35 |
| Flow (mL/min) | 2.0 | 1.0 | 1.0 |
| | | | Gradient as follows: |
| | | | 0.01min |
| | | | 3.00 min |
| | | | 10.00 min |
| | | | 11.00 min |
| | | | 12.00 min |

| **Conditions** | **Terconazole** | **Dextran sulfate** | **Raloxifene HCl** | **Clomiphene citrate** |
|---|---|---|---|---|
| Column | Zorbax C18 150x4.6mm, 3.5µm | Shodex SB802.5HQ OH pak | SymmetryShield RP18 250x4.6mm, 5µm | Symmetry C4 250x4.6mm, 5µm |
| Detection λₙₘ(nm) | 225 | By Evaporative Light Scattering Detector (ELSD)*¹ | 254 | 233 |
| Inj Vol (µL) | 200.0 | 100.0 | 10.0 | 50.0 |
| Mobile Phase | 0.1%TEA: 50mM Ammonium Acetate 70:30 | 100% DI Water | KH₂PO₄:MeCN 55:45 (pH 3) | Water:MeOH: TEA 45:55:0.3 (pH 2.5) |
| Flow (mL/min) | 1.0 | 2.0 | 1.0 | 1.0 |

| | | | | |
|---|---|---|---|---|
| *¹Conditions for ELSD - Impactor on; Evaporator drift tube temperature - 40°C; Nebuliser gas flow rate - 1.5ml/min | | | | |

### BSA method.

The dissolution samples for BSA were analysed using a BCA Protein Assay Reagent Kit. Briefly, 25µl of each sample added to 96 well plate. To this 200µl of working reagent added. The samples were incubated at 37°C for 30 minutes and then kept at room temperature for 4 hours. Samples were analysed using a Biolise Plate Reader at 570nm.

| **Conditions** | **Sumatriptan succinate** | **Tamoxifen citrate** | **Desmopressin acetate** | **Metronidazole** |
|---|---|---|---|---|
| Column | Novapak C18 150x3.9mm, 5µm | SymmetryShield RP18 250x4.6mm, 5µm | Symmetry C8 150x4.6mm, 5µm | Spherisorb ODS1 200x4.6 mm 10µm |
| Detection λₙₘ (nm) | 228 | 254 | 240 | 315 |
| Inj Vol (µL) | 10.0 | 10.0 | 200.0 | 10 |
| Mobile Phase | KH₂PO₄ (pH 5.4):MeCN 84:16 | KH₂PO₄:MeCN 55:45 (pH 3) | KH₂PO₄:MeCN 80:20 (pH 7.0) | KH₂PO₄: MeOH 70:30 |
| Flow (mL/min) | 1.0 | 1.0 | 1.0 | 1.0 |

| | | | | |
|---|---|---|---|---|
| MeCN = acetonitrile MeOH = methanol TEA = triethylamine THF = tetrahydrofuran KH₂PO₄ = potassium dihydrogen phosphate | | | | |

It will be readily appreciated by those skilled in the art that the release rates and release amounts demonstrated in the following Examples are not restrictive and can be manipulated to alter the release rate and/or release amount, as desired, by, for example, changing the loading of active in the reservoir; changing the loading of release-enhancing excipient in the reservoir; changing the number or size of the holes or openings in the sheath; and/or changing the dimensions of the reservoir and/or the sheath; or a mixture of some or all of these parameters.

### Example 1 (Influence of Number of Holes)

Intravaginal drug delivery devices in the form of a "core" design ring having a full length (140mm) 10% (w/w) metronidazole reservoir (total drug content ∼400 mg metronidazole) and either 0, 4 or 8 holes on the outer surface of the device were prepared by following the General Method of Manufacture for Examples 1-4.

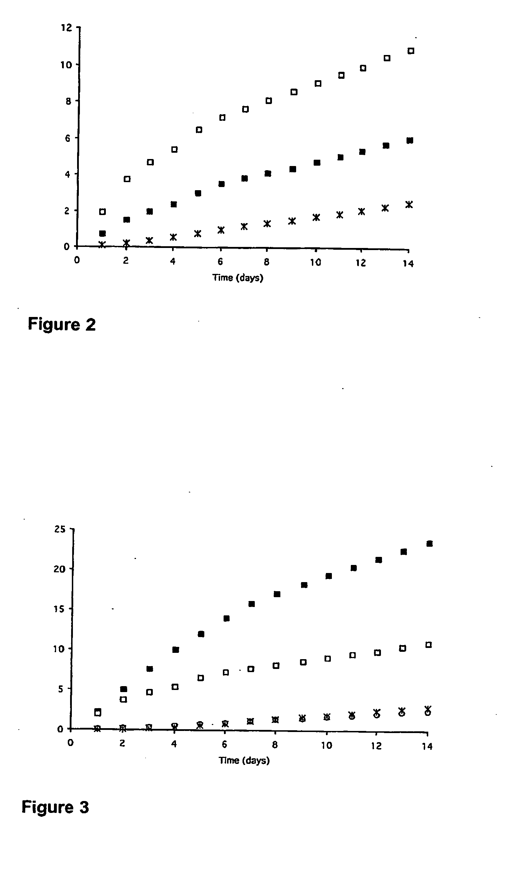

The influence of the number of holes on the cumulative *in vitro* metronidazole release from the rings is illustrated in Figure 2. Increasing the number of holes leads to an increase in the daily release rate, such that, after 14 days, the cumulative amounts released from the 0, 4 and 8 hole rings are 2.5, 6.0 and 10.9 mg, respectively.

### Example 2 (Influence of number of holes and drug loading)

Intravaginal drug delivery devices in the form of a reservoir design ring having a full length 20% (w/w) metronidazole reservoir (total drug content ∼800 mg metronidazole) and either 0 or 8 holes on the outer surface of the device were prepared by following the General Method of Manufacture for Examples 1-4.

The influence of reservoir drug loading and number of holes on the cumulative *in vitro* metronidazole release from the rings is illustrated in Figure 3.

For the rings with no holes, the release profiles for the 10% and 20% (w/w) metronidazole-loaded rings are similar to each other, since it is the sheath which is controlling the release rate. Specifically, after 14 days, the cumulative amounts released from the 0 and 8 hole rings are 2.5 and 2.9 mg, respectively.

However, for the rings with an identical number and size of holes (8), release then becomes a function of drug loading. Increasing the reservoir metronidazole concentration from 10 to 20% (w/w) leads to an increase in the daily release rate, such that, after 14 days, the cumulative amounts released from the 10% and 20% (w/w) rings are 10.9 and 23.5 mg, respectively.

### Example 3 (Influence of addition of pore-forming excipient to drug loaded reservoir)

Intravaginal drug delivery devices in the form of a reservoir design ring having a full length, 10% (w/w) metronidazole plus 30% (w/w) hydroxyethylcellulose (HEC)-loaded reservoir (~400 mg metronidazole reservoir content plus ~1200 mg HEC reservoir content) and either 0 or 8 holes on the outer surface of the device were prepared by following the General Method of Manufacture for Examples 1-4.

The influence of incorporating hydroxyethylcellulose, a hydrophilic pharmaceutical excipient, and the number of holes is illustrated in Figure 4.

For the ring with no holes, after 14 days, the cumulative amount released remains at 2.5 mg. The incorporation of 10% metronidazole, without or with, 30% (w/w) hydroxyethylcellulose into rings each having 8 holes leads to a significant increase in the amount of metronidazole released, such that, after 14 days, the cumulative amounts released from 0 and 30% (w/w) HEC-loaded reservoir rings are 10.9 and 54.9 mg, respectively.

### Example 4

Intravaginal drug delivery devices in the form of a reservoir design ring having a full length 5% (w/w) metronidazole-loaded reservoir (~200 mg metronidazole reservoir content) and either 0 or 8 holes on the outer or inner surface of the device were prepared by following the General Method of Manufacture for Examples 1-4.

Figure 5 demonstrates that the amount of metronidazole released *in vitro* from a 5% (w/w) metronidazole-loaded reservoir intravaginal ring is not dependent upon the location of the holes on the device surface. The release profiles for rings having holes on the external and internal surfaces are similar. Specifically, after 11 days, the cumulative amounts released from 8 (internal or external) hole rings are 7.4 and 7.5 mg, respectively.

### Example 5 : Fluoxetine hydrochloride

Intravaginal drug delivery devices, in duplicate, in the form of a reservoir design ring having a full length, 30% (w/w) fluoxetine hydrochloride-containing reservoir and either 0 or 8 holes on the outer surface of the device were prepared by following the General Method of Manufacture for Examples 5-8.

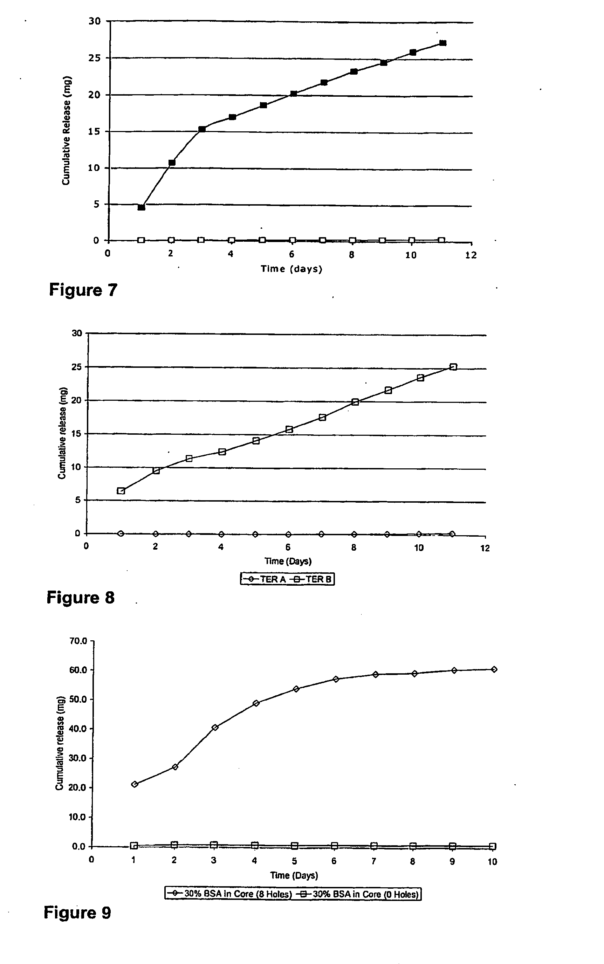

Release data are shown in Fig. 7 and in the table below:

| Time (days) | Cumulative Release Perforated Ring (mg) | Cumulative Release Reservoir Ring (mg) |
|---|---|---|
| 1 | 4.537 | 0.030 |
| 2 | 10.704 | 0.066 |
| 3 | 15.252 | 0.093 |
| 4 | 17.003 | 0.122 |
| 5 | 18.625 | 0.149 |
| 6 | 20.225 | 0.178 |
| 7 | 21.769 | 0.205 |
| 8 | 23.310 | 0.231 |
| 9 | 24.537 | 0.258 |
| 10 | 25.976 | 0.285 |
| 11 | 27.214 | 0.285 |

### Example 6: Terconazole

Intravaginal drug delivery devices, in duplicate, in the form of a reservoir design ring having a full length, 30% (w/w) terconazole-containing reservoir and either 0 or 8 holes on the outer surface of the device were prepared by following the General Method of Manufacture for Examples 5-8.

Release data are shown in Fig. 8 and in the table below:

| Time (days) | Cumulative Release Reservoir Ring (mg) | Cumulative Release Perforated Ring (mg) |
|---|---|---|
| 1 | 0.0019 | 6.3794 |
| 2 | 0.0027 | 9.5309 |
| 3 | 0.0057 | 11.342 |
| 4 | 0.0258 | 12.3921 |
| 5 | 0.0558 | 13.8321 |
| 6 | 0.0958 | 15.6321 |
| 7 | 0.1458 | 17.3921 |
| 8 | 0.149 | 19.7108 |
| 9 | 0.1775 | 21.5015 |
| 10 | 0.2311 | 23.3955 |
| 11 | 0.3025 | 25.0568 |

### Example 7 : Bovine Serum Albumin

Intravaginal drug delivery devices, in duplicate, in the form of a reservoir design ring having a full length, 30% (w/w) bovine serim albumin-containing reservoir and either 0 or 8 holes on the outer surface of the device were prepared by following the General Method of Manufacture for Examples 5-8. Bovine serum albumin (Molecular weight - 66kD) was chosen to demonstrate that large peptides/ large proteins can be released from the perforated drug delivery device of the present invention.

Release data are shown in Fig. 9 and in the table below:

| Time (Days) | Cumulative Release Perforated Ring (mg) | Cumulative Release Reservoir Ring (mg) |
|---|---|---|
| 1 | 21.22 | 0.46 |
| 2 | 27.19 | 0.75 |
| 3 | 40.74 | 0.77 |
| 4 | 49.16 | 0.78 |
| 5 | 54.05 | 0.79 |
| 6 | 57.42 | 0.79 |
| 7 | 59.04 | 0.79 |
| 8 | 59.43 | 0.79 |
| 9 | 60.60 | 0.79 |
| 10 | 61.10 | 0.79 |

### Example 8 : Dextran Sulphate

Intravaginal drug delivery devices, in duplicate, in the form of a reservoir design ring having a full length, 30% (w/w) dextran sulphate-containing reservoir and either 0 or 8 holes on the outer surface of the device were prepared by following the General Method of Manufacture for Examples 5-8. Dextran sulphate was, again, chosen to demonstrate that large carbohydrates can be released from the perforated drug delivery device of the present invention.

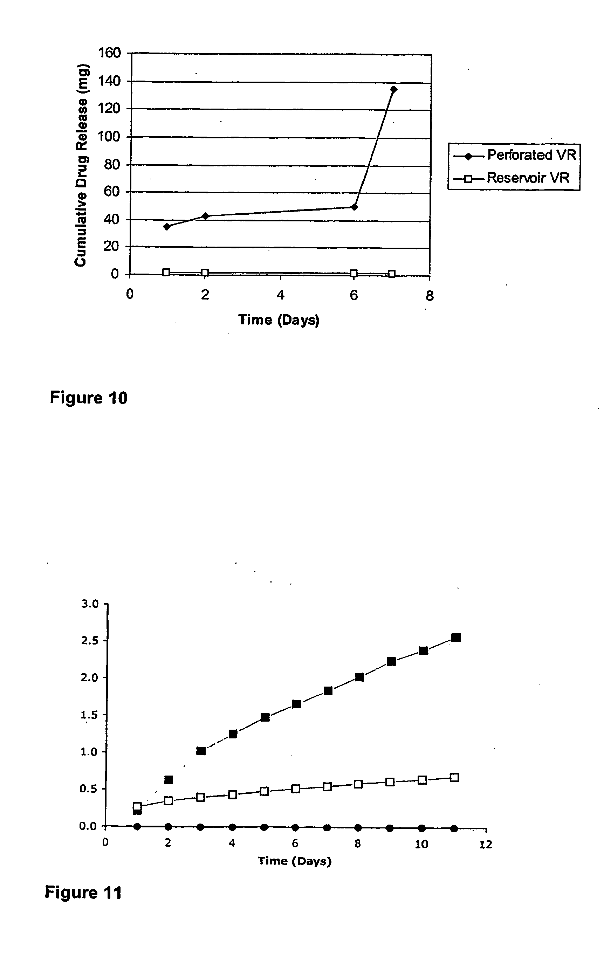

Release data are shown in Fig. 10 and in the table below:

| Time (Days) | Cumulative Release Perforated Ring (mg) | Cumulative Release Reservoir Ring (mg) |
|---|---|---|
| 1 | 35.06 | 1.31 |
| 2 | 42.97 | 1.31 |
| 6 | 49.80 | 1.31 |
| 7 | 135.03 | 1.31 |

### Example 9: Leuprolide Acetate

Intravaginal drug delivery devices, in duplicate, in the form of a reservoir design ring having a full length, 5% (w/w) leuprolide-containing reservoir and either 0 or 8 holes or one slit (dimension: 25mm X 4mm) on the outer surface of the device were prepared by following the General Method of Manufacture for Examples 9-14.

Release data are shown in Fig. 11 and in the table below:

| Day | Cumulative Release Perforated Ring (mg) | Cumulative Release Reservoir Ring (mg) | Cumulative Release Slitted Ring (mg) |
|---|---|---|---|
| 1 | 0.216 | 0 | 0.267 |
| 2 | 0.625 | 0 | 0.343 |
| 3 | 1.020 | 0 | 0.394 |
| 4 | 1.251 | 0 | 0.437 |
| 5 | 1.476 | 0 | 0.479 |
| 6 | 1.661 | 0 | 0.516 |
| 7 | 1.844 | 0 | 0.546 |
| 8 | 2.027 | 0 | 0.585 |
| 9 | 2.244 | 0 | 0.618 |
| 10 | 2.390 | 0 | 0.645 |
| 11 | 2.572 | 0 | 0.680 |

### Example 10 :Desmopressin Acetate

Intravaginal drug delivery devices, in duplicate, in the form of a reservoir design ring having a full length, 5% (w/w) desmopressin-containing reservoir and either 0 or 8 holes on the outer surface of the device were prepared by following the General Method of Manufacture for Examples 9-14.

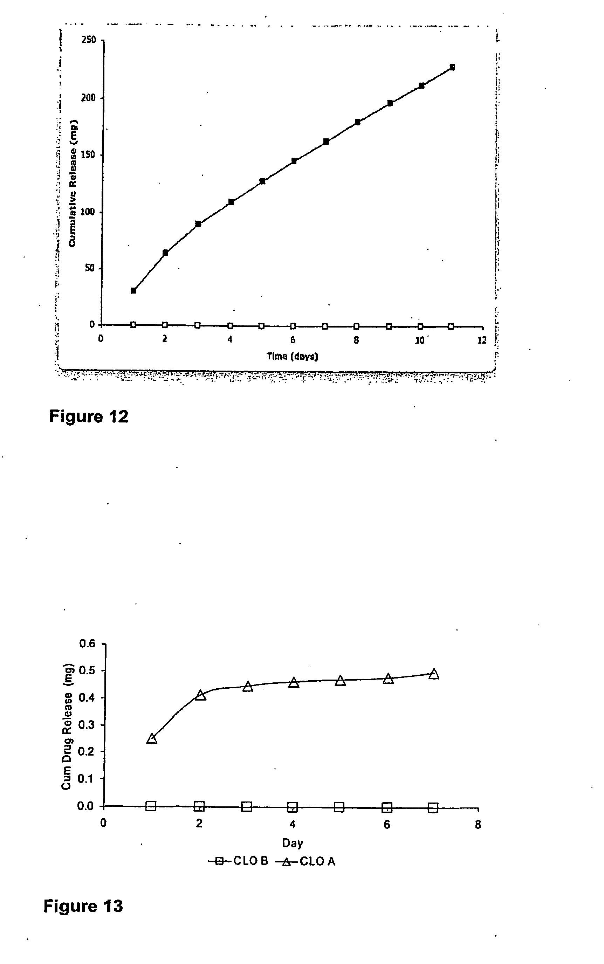

Release data are shown in Fig. 12 and in table below:

| Day | Cumulative Release Perforated Ring (mg) | Cumulative Release Reservoir Ring (mg) |
|---|---|---|
| 1 | 30.2 | 0 |
| 2 | 64.4 | 0 |
| 3 | 89.9 | 0 |
| 4 | 110.1 | 0 |
| 5 | 128.5 | 0 |
| 6 | 146.3 | 0 |
| 7 | 163.5 | 0 |
| 8 | 180.3 | 0 |
| 9 | 196.9 | 0 |
| 10 | 212.6 | 0 |
| 11 | 228.3 | 0 |

### Example 11 : Clomiphene Citrate

Intravaginal drug delivery devices, in duplicate, in the form of a reservoir design ring having a full length, 5% (w/w) clomiphene-containing reservoir and either 0 or 8 holes on the outer surface of the device were prepared by following the General Method of Manufacture for Examples 9-14.

Release data are shown in Fig. 13 and in the table below:

| Time (Days) | Cumulative Release Reservoir Ring (mg) | Cumulative Release Perforated Ring (mg) |
|---|---|---|
| 1 | 0 | 0.251 |
| 2 | 0 | 0.411 |
| 3 | 0 | 0.447 |
| 4 | 0 | 0.462 |
| 5 | 0 | 0.470 |
| 6 | 0 | 0.478 |
| 7 | 0 | 0.496 |

### Example 12 : Raloxifene HCl

Intravaginal drug delivery devices, in duplicate, in the form of a reservoir design ring having a full length, 5% (w/w) raloxifene-containing reservoir and either 0 or 8 holes on the outer surface of the device were prepared by following the General Method of Manufacture for Examples 9-14.

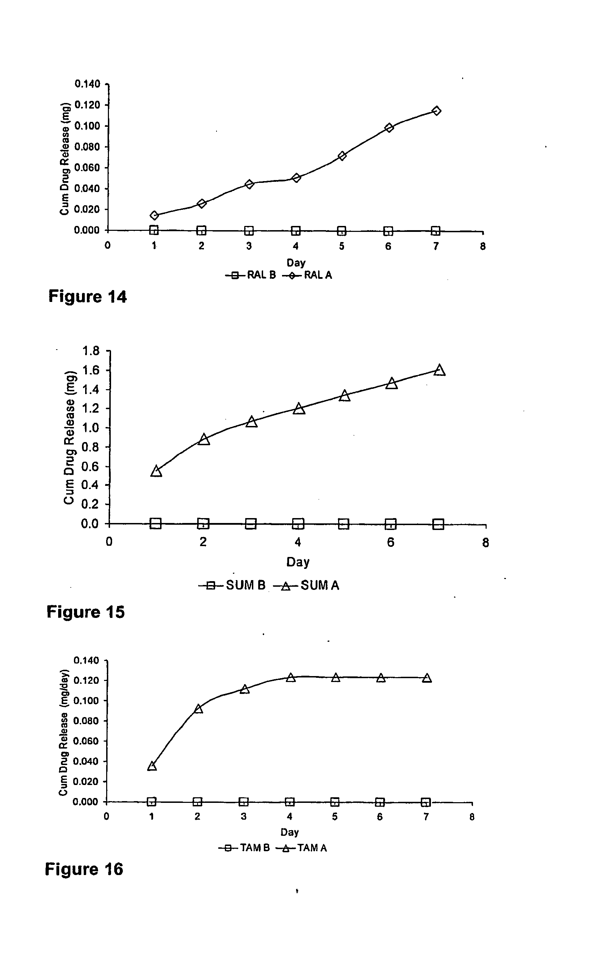

Release data are shown in Fig. 14 and in the table below:

| Time (Days) | Cumulative Release Reservoir Ring (mg) | Cumulative Release Perforated Ring (mg) |
|---|---|---|
| 1 | 0 | 0.014 |
| 2 | 0 | 0.026 |
| 3 | 0 | 0.045 |
| 4 | 0 | 0.051 |
| 5 | 0 | 0.072 |
| 6 | 0 | 0.100 |
| 7 | 0 | 0.115 |

### Example 13 : Sumatriptan Succinate

Intravaginal drug delivery devices, in duplicate, in the form of a reservoir design ring having a full length, 5% (w/w) sumatriptan-containing reservoir and either 0 or 8 holes on the outer surface of the device were prepared by following the General Method of Manufacture for Examples 9-14.

Release data are shown in Fig. 15 and in the table below:

| Time (Days) | Cumulative Release Reservoir Ring (mg) | Cumulative Release Perforated Ring (mg) |
|---|---|---|
| 1 | 0 | 0.555 |
| 2 | 0 | 0.884 |
| 3 | 0 | 1.077 |
| 4 | 0 | 1.213 |
| 5 | 0 | 1.352 |
| 6 | 0 | 1.480 |
| 7 | 0 | 1.611 |

### Example 14 : Tamoxifen Citrate

Intravaginal drug delivery devices, in duplicate, in the form of a reservoir design ring having a full length, 5% (w/w) tamoxifen-containing reservoir and either 0 or 8 holes on the outer surface of the device were prepared by following the General Method of Manufacture for Examples 9-14.

Release data are shown in Fig. 16 and in the table below:

| Time (Days) | Cumulative Release Reservoir Ring (mg) | Cumulative Release Perforated Ring (mg) |
|---|---|---|
| 1 | 0 | 0.036 |
| 2 | 0 | 0.092 |
| 3 | 0 | 0.112 |
| 4 | 0 | 0.124 |
| 5 | 0 | 0.124 |
| 6 | 0 | 0.124 |
| 7 | 0 | 0.124 |

### Example 15 : Fluoxetine HCL

Gel-filled intravaginal drug delivery devices in the form of rings have been manufactured containing 5% w/w fluoxetine HCl in a 3% w/w hydroxyethylcellulose (HEC) aqueous gel base. A series of holes was punched in a length of transparent, medical-grade silicone tubing, and the ends of the tube joined to form a torus. The gel was then syringed into the core of the tube through one of the holes. As the release study has progressed, we have been able to observe the 'gel depletion front' regress away from the holes, confirming release is taking place.

A semi-solid gel reservoir formulation containing fluoxetine HCL was prepared having the following composition:
3.0% w/w hydroxyethyl cellulose 250 HHX-Pharm
5.0% w/w fluoxetine hydrochloride (micronised)
92.0% w/w deionised water

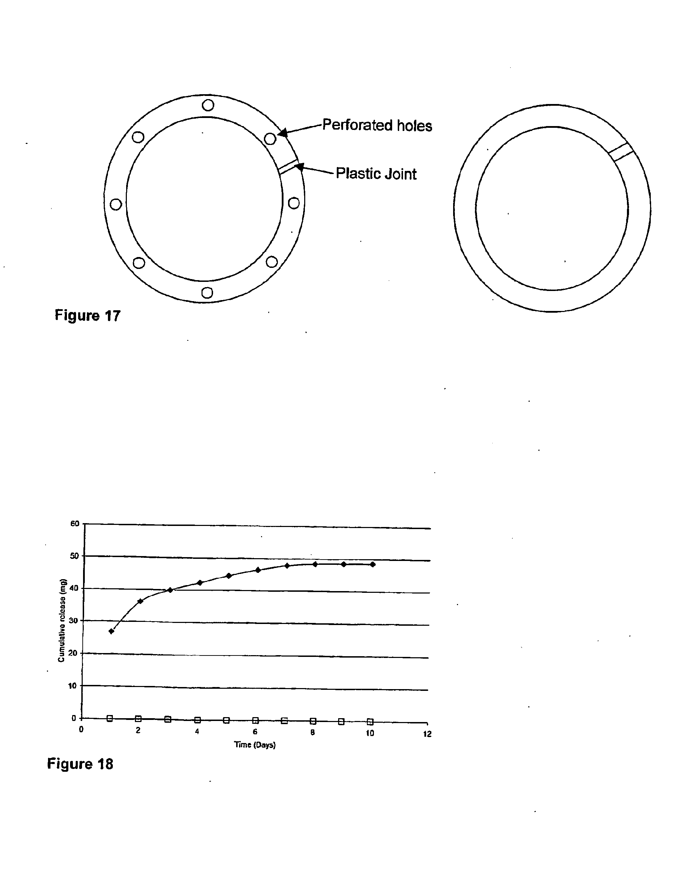

Medical-grade silicone tubing (5.8mm outer diameter, 3.0mm inner diameter) was cut into lengths of 176mm (equivalent to a circumference of 7.6mm x 56 mm intravaginal ring). For manufacture of non-perforated gel-filled rings, 1.0g of the fluoxetine HCL gel formulation was injected via the open ends so as to fill the length of tubing, and the ends of the tube subsequently joined with a plastic plug (Figure 17). For the manufacture of perforated gel-filled rings, the length of medical grade silicone was first perforated, using a size 1 cork borer (diameter of holes - 4mm), with eight holes located at regular intervals along the tube length, before injection of the 1.0g of fluoxetine HCL gel formulation and joining of tube ends.

Duplicate non-perforated and perforated rings were placed in conical flasks containing 50 ml of 0.9% saline solution and maintained at 37.0 deg C in an orbital incubator (60 rpm). The release medium was sampled daily over a ten day period with complete daily replacement of the release medium.

The results, presented in Figure 18 and the table below, demonstrate enhanced release from the perforated gel-filled vaginal ring devices. In terms of fractional release:
- fluoxetine gel-filled perforated rings released 95-98% w/w of total active content
- fluoxetine gel-filled non-perforated rings released 0.2% w/w of total active content

| Time (days) | Cumulative Release Perforated Reservoir Gel Ring (mg) | Cumulative Release Reservoir Gel Ring (mg) |
|---|---|---|
| 1 | 26.848 | 0.044 |
| 2 | 36.249 | 0.059 |
| 3 | 39.924 | 0.070 |
| 4 | 42.297 | 0.079 |
| 5 | 44.545 | 0.086 |
| 6 | 46.402 | 0.093 |
| 7 | 47.826 | 0.098 |
| 8 | 48.422 | 0.101 |
| 9 | 48.538 | 0.103 |
| 10 | 48.592 | 0.104 |

### Example 16 : Acyclovir Rod - type silicone devices

Rod-type devices containing acyclovir were manufactured to determine the rate of drug release in the presence or absence of a crosslinked excipient, namely croscarmellose (CCM). The rod-type devices, for use in the present Example and following Example 17, were manufactured using the following method:

**Table 1. Composition of formulation mix - Example 16**

| % w/w Croscarmellose | % w/w Acyclovir | % w/w PDMS (10% w/w Filler) |
|---|---|---|
| 0 | 10 | 90 |
| 10 | 10 | 80 |
| 20 | 10 | 70 |
| 30 | 10 | 60 |

Catalyst (1% w/w) was added to each formulation which was then injected into medical grade silicone tubing (5.8mm outer diameter, 3.0 mm inner diameter). The tubing was then cut into 15.0mm long samples and put on dissolution at 37 degrees C(10 ml deionised water). Three replicates of each were performed.

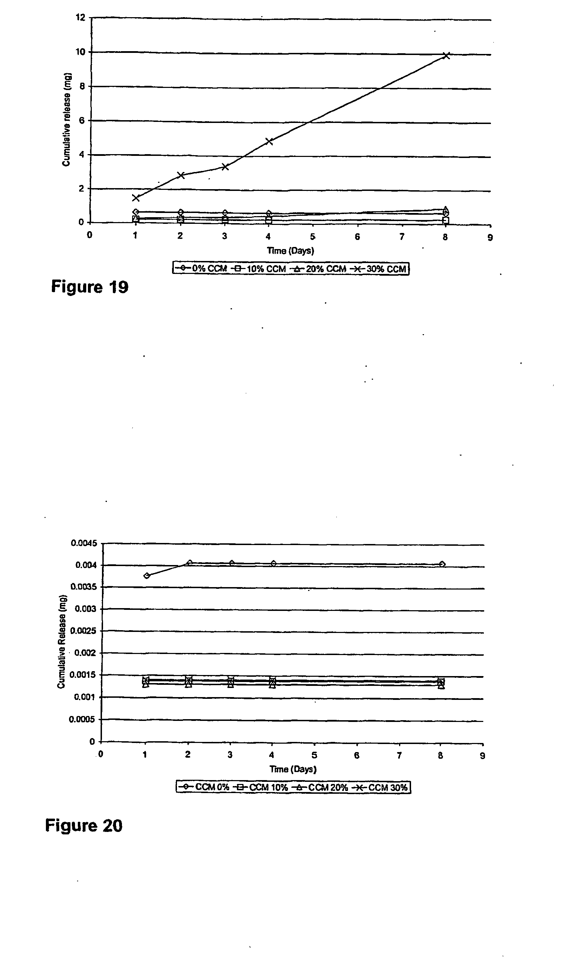

The cumulative release data are shown in Fig. 19 and in the table below:

| Time (Days) | 0% CCM | 10% CCM | 20% CCM | 30% CCM |
|---|---|---|---|---|
| 1 | 0.632 | 0.212 | 0.267 | 1.471 |
| 2 | 0.635 | 0.222 | 0.329 | 2.819 |
| 3 | 0.636 | 0.225 | 0.357 | 3.367 |
| 4 | 0.637 | 0.230 | 0.452 | 4.892 |
| 8 | 0.637 | 0.245 | 0.901 | 9.908 |

### Example 17 : Leuprolide Acetate rod - type silicone devices.

Rod-type devices containing leuprolide acetate were manufactured to determine the rate of drug release in the presence or absence of a crosslinked excipient. The rod-type devices were manufactured using the following method:

**Table 1. Composition of formulation mix - Example 17**

| Formulation Code | % w/w Croscarmellose | % w/w Leuprolide acetate | % w/w PDMS (10% w/w Filler) |
|---|---|---|---|
| 0% CCM | 0 | 1 | 99 |
| 10% CCM | 10 | 1 | 89 |
| 20% CCM | 20 | 1 | 79 |
| 30% CCM | 30 | 1 | 69 |

Catalyst (1% w/w) was added to each formulation which was then injected into medical grade silicone tubing (5.8mm OD, 3.0 mmID). The tubing was then cut into 15.0mm long samples and put on dissolution at 37 degreesC (10 ml deionised water). Three replicates of each were performed.

The cumulative release data are shown on Fig. 20 and in the table below:

| Time (Days) | 0% CCM | 10% CCM | 20% CCM | 30% CCM |
|---|---|---|---|---|
| 1 | 0.00376 | 0.00138 | 0.00130 | 0.00140 |
| 2 | 0.00406 | 0.00138 | 0.00130 | 0.00140 |
| 3 | 0.00406 | 0.00138 | 0.00130 | 0.00140 |
| 4 | 0.00406 | 0.00138 | 0.00130 | 0.00140 |
| 8 | 0.00406 | 0.00138 | 0.00130 | 0.00140 |

## Claims

1. An intravaginal drug delivery device for administration into a vaginal environment, the device comprising at least one reservoir, the, or each, reservoir containing at least one pharmacologically active agent or a prodrug thereof, dispersed in a hydrophobic elastomeric polymer; and a sheath discontinuously surrounding the at least one reservoir, so that, in use, at least part of the at least one reservoir is directly exposed to the vaginal environment.

2. An intravaginal drug delivery device according to Claim 1, in which the sheath defines one or more holes or openings, the, or each, hole or opening extending through the sheath to the at least one reservoir, so that at least part of the at least one reservoir is exposed, in use, to the vaginal environment.

3. An intravaginal drug delivery device according to Claim 2, in which the, or each, hole or opening may extend to the surface of the at least one reservoir or may, in addition, extend at least partially into the at least one reservoir.

4. An intravaginal drug delivery device according to Claim 2 or 3, in which the, or each, hole or opening may be of any shape or may be joined with an adjacent hole or opening to give a continuous opening in the form of a slit.

5. An intravaginal drug delivery device according to Claim 2 or 3, in which the, or each, hole or opening is substantially cylindrical with a diameter in the range of about 0.5 to 6.5 mm, preferably about 1 to 5 mm.

6. An intravaginal drug delivery device according to any one of Claims 2-5, in which the, or each, hole or opening may extend through the sheath substantially normal to the reservoir surface.

7. An intravaginal drug delivery device according to any one of Claims 2-6, in which the device is substantially circular in transverse cross-section, and the, or each, hole extends substantially radially, inwardly or outwardly, through the sheath.

8. An intravaginal drug delivery device according to Claim 7, in which there are one to thirty, optionally two to ten, further optionally three to ten, of said holes, optionally aligned linearly, along the inner or outer circumference, optionally the inner circumference, of the intravaginal drug delivery device.

9. An intravaginal drug delivery device according to any one of Claims 2-6, in which the device is a substantially cylindrical rod device, and said holes are provided at each terminal end of the rod.

10. An intravaginal drug delivery device according to Claim 9, in which the rod device defines a right circular cylinder and each base of the rod is partly or fully exposed, to define said holes.

11. An intravaginal drug delivery device according to Claim 9 or 10, in which further holes or slits are provided extending substantially radially through the sheath.

12. An intravaginal drug delivery device according to Claim 11, in which there are one to thirty, optionally two to ten, further optionally three to ten, of said further holes, optionally aligned linearly, along the circumference of the rod.

13. An intravaginal drug delivery device according to any one of Claims 1-8, in which the device is a partial or complete toroid shape, preferably a partial or complete torus shape.

14. An intravaginal drug delivery device according to any one of the preceding claims, in which the reservoir additionally comprises at least one pore-forming excipient; in which the pore-forming excipient, optionally, comprises a water-soluble or water-swellable polysaccharide, preferably a cellulose derivative, more preferably hydroxyethylcellulose or croscarmellose; a monosaccharide or a disaccharide, preferably glucose or lactose; a water-soluble salt; a protein, preferably a gelatin; a nonionic surface active agent; a bile salt; an organic solvent, preferably ethoxydiglycol or polyethylene glycol; or a fatty acid ester, preferably containing 2 to 20 carbon atoms, more preferably a myristate ester.

15. An intravaginal drug delivery device according to any one of the preceding claims, in which the sheath additionally comprises at least one pharmacologically active agent

16. A method of manufacturing an intravaginal drug delivery device according to any one of the preceding claims, said method comprising the steps of forming a reservoir by dispersing at least one pharmacologically active agent in a pharmaceutically acceptable hydrophobic elastomeric polymer; curing the reservoir; and applying a sheath to partly surround the reservoir.

17. A method of manufacturing an intravaginal drug delivery device according to any one of Claims 1 to 15, said method comprising injecting or extruding a reservoir material into a hollow sheath.

18. An intravaginal drug delivery device according to any one of Claims 1 to 15 or a method according to Claim 16 or 17 of manufacturing an intravaginal drug delivery device according to any one of Claims 1 to 15, in which the hydrophobic elastomeric polymer is selected from the room-temperature vulcanizing type of organopolysiloxanes, poly(ethylene-co-vinyl acetate) and styrene-butadiene-styrene block copolymer.

## Patentansprüche

1. Intravaginale Arzneimittelabgabevorrichtung zur Verabreichung in eine vaginale Umgebung, wobei die Vorrichtung Folgendes umfasst: mindestens ein Reservoir, wobei das oder jedes Reservoir mindestens einen pharmakologischen Wirkstoff oder ein Prodrug davon enthält, der/das in einem hydrophoben elastomeren Polymer dispergiert ist; und eine Hülle, die das mindestens eine Reservoir in unterbrochener Weise umgibt, sodass bei Verwendung zumindest ein Teil des mindestens einen Reservoirs der vaginalen Umgebung direkt ausgesetzt ist.

2. Intravaginale Arzneimittelabgabevorrichtung nach Anspruch 1, in der die Hülle ein/e oder mehrere Löcher oder Öffnungen definiert, wobei sich das/die oder jedes/jede Loch oder Öffnung durch die Hülle bis zum mindestens einen Reservoir erstreckt, sodass zumindest ein Teil des mindestens einen Reservoirs bei Verwendung der vaginalen Umgebung ausgesetzt ist.

3. Intravaginale Arzneimittelabgabevorrichtung nach Anspruch 2, in der sich das/die oder jedes/jede Loch oder Öffnung bis zur Oberfläche des mindestens einen Reservoirs erstrecken kann oder sich zusätzlich zumindest teilweise bis in das mindestens eine Reservoir erstrecken kann.

4. Intravaginale Arzneimittelabgabevorrichtung nach Anspruch 2 oder 3, in der das/die oder jedes/jede Loch oder Öffnung eine jedwede Form haben kann oder mit einem/einer benachbarten Loch oder Öffnung verbunden sein kann, um eine kontinuierliche Öffnung in Form eines Schlitzes zu bilden.

5. Intravaginale Arzneimittelabgabevorrichtung nach Anspruch 2 oder 3, in der das/die oder jedes/jede Loch oder Öffnung im Wesentlichen zylindrisch ist, mit einem Durchmesser im Bereich von etwa 0,5 bis 6,5 mm, bevorzugt etwa 1 bis 5 mm.

6. Intravaginale Arzneimittelabgabevorrichtung nach einem der Ansprüche 2-5, in der sich das/die oder jedes/jede Loch oder Öffnung durch die Hülle im Wesentlichen senkrecht zur Reservoiroberfläche erstreckt.

7. Intravaginale Arzneimittelabgabevorrichtung nach einem der Ansprüche 2-6, in der die Vorrichtung im Wesentlichen kreisförmig im Querschnitt ist und sich das oder jedes Loch im Wesentlichen strahlenförmig, nach innen oder nach außen, durch die Hülle erstreckt.

8. Intravaginale Arzneimittelabgabevorrichtung nach Anspruch 7, in der sich ein bis dreißig, optional zwei bis zehn, weiterhin optional drei bis zehn, der genannten Löcher befinden, die optional linear, entlang des inneren oder äußeren Umfangs, optional des inneren Umfangs, der intravaginalen Arzneimittelabgabevorrichtung angeordnet sind.

9. Intravaginale Arzneimittelabgabevorrichtung nach einem der Ansprüche 2-6, in der die Vorrichtung eine im Wesentlichen zylindrische Stabvorrichtung ist und die genannten Löcher an jedem terminalen Ende des Stabs bereitgestellt sind.

10. Intravaginale Arzneimittelabgabevorrichtung nach Anspruch 9, in der die Stabvorrichtung einen geraden Kreiszylinder definiert und jede Grundfläche des Stabs teilweise oder vollständig ausgesetzt ist, um die genannten Löcher zu definieren.

11. Intravaginale Arzneimittelabgabevorrichtung nach Anspruch 9 oder 10, in der weitere Löcher oder Schlitze bereitgestellt sind, die sich im Wesentlichen strahlenförmig durch die Hülle erstrecken.

12. Intravaginale Arzneimittelabgabevorrichtung nach Anspruch 11, in der sich ein bis dreißig, optional zwei bis zehn, weiterhin optional drei bis zehn, der genannten weiteren Löcher befinden, die optional linear, entlang des Umfangs des Stabs angeordnet sind.

13. Intravaginale Arzneimittelabgabevorrichtung nach einem der Ansprüche 1-8, in der die Vorrichtung eine teilweise oder vollständige toroidale Form, bevorzugt eine teilweise oder vollständige Torusform hat.

14. Intravaginale Arzneimittelabgabevorrichtung nach einem der vorstehenden Ansprüche, in der das Reservoir zusätzlich mindestens einen porenbildenden Hilfsstoff umfasst; in dem der porenbildende Hilfsstoff optional Folgende umfasst: ein wasserlösliches oder mit Wasser quellbares Polysaccharid, bevorzugt ein Cellulosederivat, bevorzugter Hydroxyethylcellulose oder Croscarmellose; ein Monosaccharid oder ein Disaccharid, bevorzugt Glucose oder Lactose; ein wasserlösliches Salz; ein Protein, bevorzugt eine Gelatine; einen nichtionischen oberflächenaktiven Stoff; ein Gallensalz; ein organisches Lösungsmittel, bevorzugt Ethoxydiglycol oder Polyethylenglycol; oder einen Fettsäureester, der bevorzugt 2 bis 20 Kohlenstoffatome enthält, bevorzugter ein Myristatester ist.

15. Intravaginale Arzneimittelabgabevorrichtung nach einem der vorstehenden Ansprüche, in der die Hülle zusätzlich mindestens einen pharmakologischen Wirkstoff umfasst.

16. Verfahren zur Herstellung einer intravaginalen Arzneimittelabgabevorrichtung nach einem der vorstehenden Ansprüche, wobei das genannte Verfahren die folgenden Schritte umfasst: Bilden eines Reservoirs durch Dispergieren von mindestens einem pharmakologischen Wirkstoff in einem pharmazeutisch verträglichen hydrophoben elastomeren Polymer; Aushärten des Reservoirs; und Anbringen einer Hülle, um das Reservoir teilweise zu umgeben.

17. Verfahren zur Herstellung einer intravaginalen Arzneimittelabgabevorrichtung nach einem der Ansprüche 1 bis 15, wobei das genannte Verfahren das Einspritzen oder Extrudieren eines Reservoirmaterials in eine hohle Hülle umfasst.

18. Intravaginale Arzneimittelabgabevorrichtung nach einem der Ansprüche 1 bis 15 oder ein Verfahren nach Anspruch 16 oder 17 zur Herstellung einer intravaginalen Arzneimittelabgabevorrichtung nach einem der Ansprüche 1 bis 15, in der/dem das hydrophobe elastomere Polymer aus dem Raumtemperatur-vulkanisierenden Typ von Organopolysiloxanen, Poly(ethylen-co-vinylacetat) und Styrol-Butadien-Styrol-Blockcopolymer ausgewählt ist.

## Revendications

1. Dispositif de libération intravaginale d'un médicament à être administré dans l'environnement vaginal, le dispositif comprenant au moins un réservoir, le ou chaque réservoir contenant au moins un agent pharmacologiquement actif ou un précurseur de celui-ci dispersé dans un polymère élastomère hydrophobe ; et une gaine entourant de manière discontinue le au moins un réservoir de telle façon qu'en cours d'utilisation, une partie au moins du au moins un réservoir est directement exposée à l'environnement vaginal.

2. Dispositif de libération intravaginale d'un médicament selon la revendication 1, dans lequel la gaine définit une ou plusieurs perforations ou ouvertures, la ou chaque perforation ou ouverture s'étendant au travers de la gaine jusqu'au au moins un réservoir de telle façon qu'en cours d'utilisation, une partie au moins du au moins un réservoir est exposée à l'environnement vaginal.

3. Dispositif de libération intravaginale d'un médicament selon la revendication 2, dans lequel la ou chaque perforation ou ouverture peut s'étendre jusqu'à la surface du au moins un réservoir ou peut également s'étendre au moins en partie dans le au moins un réservoir.

4. Dispositif de libération intravaginale d'un médicament selon la revendication 2 ou 3, dans lequel la ou chaque perforation ou ouverture peut avoir toute forme ou peut être jointe à une perforation ou ouverture adjacente pour produire une ouverture continue en forme de fente.

5. Dispositif de libération intravaginale d'un médicament selon la revendication 2 ou 3, dans lequel la ou chaque perforation ou ouverture a une forme globalement cylindrique dont le diamètre va d'environ 0,5 à 6,5 mm, et qui est préférablement d'environ 1 à 5 mm.

6. Dispositif de libération intravaginale d'un médicament selon l'une quelconque des revendications 2-5, dans lequel la ou chaque perforation ou ouverture peut s'étendre au travers de la gaine d'une manière globalement perpendiculaire à la surface du réservoir.

7. Dispositif de libération intravaginale d'un médicament selon l'une quelconque des revendications 2-6, dans lequel le dispositif est globalement circulaire en coupe transversale, la ou chaque perforation s'étendant d'une manière globalement radiale vers l'intérieur ou vers l'extérieur au travers de la gaine.

8. Dispositif de libération intravaginale d'un médicament selon la revendication 7, dans lequel il existe de une à trente, en option de deux à dix et également en option de trois à dix desdites perforations, en option alignées d'une manière linéaire, le long de la circonférence interne ou externe, et en option de la circonférence interne, du dispositif de libération intravaginale d'un médicament.

9. Dispositif de libération intravaginale d'un médicament selon l'une quelconque des revendications 2-6, dans lequel le dispositif est globalement un dispositif en tige cylindrique, lesdites perforations étant situées à chaque extrémité terminale de la tige.

10. Dispositif de libération intravaginale d'un médicament selon la revendication 9, dans lequel le dispositif en tige définit un cylindre circulaire droit, chaque base de la tige étant partiellement ou totalement exposée pour définir lesdites perforations.

11. Dispositif de libération intravaginale d'un médicament selon la revendication 9 ou 10, dans lequel des perforations ou fentes additionnelles, s'étendant d'une manière globalement radiale au travers de la gaine, sont prévues.

12. Dispositif de libération intravaginale d'un médicament selon la revendication 11, dans lequel il existe de une à trente, en option de deux à dix et également en option de trois à dix desdites perforations additionnelles, en option alignées de manière linéaire, le long de la circonférence de la tige.

13. Dispositif de libération intravaginale d'un médicament selon l'une quelconque des revendications 1-8, dans lequel le dispositif a une forme toroïdale partielle ou complète, et préférablement la forme d'un tore partiel ou complet.

14. Dispositif de libération intravaginale d'un médicament selon l'une quelconque des revendications précédentes, dans lequel le réservoir comprend également au moins un excipient porogène, l'excipient porogène comprenant en option un polysaccharide hydrosoluble ou gonflant au contact de l'eau, préférablement un dérivé cellulosique et plus préférablement l'hydroxyéthylcellulose ou la croscarmellose ; un monosaccharide ou un disaccharide, préférablement le glucose ou le lactose ; un sel hydrosoluble ; une protéine, préférablement une gélatine ; un agent tensio-actif non ionique ; un sel biliaire ; un solvant organique, préférablement l'éthoxydiglycol ou le polyéthylèneglycol ; ou un ester d'acide gras contenant préférablement de 2 à 20 atomes de carbone, plus préférablement un ester de myristate.

15. Dispositif de libération intravaginale d'un médicament selon l'une quelconque des revendications précédentes, dans lequel la gaine comprend également au moins un agent pharmacologiquement actif.

16. Méthode de fabrication d'un dispositif de libération intravaginale d'un médicament selon l'une quelconque des revendications précédentes, ladite méthode comprenant les étapes de formation d'un réservoir par dispersion d'au moins un agent pharmacologiquement actif dans un polymère élastomère hydrophobe pharmaceutiquement acceptable ; de cuisson du réservoir ; et d'application d'une gaine pour envelopper en partie le réservoir.

17. Méthode de fabrication d'un dispositif de libération intravaginale d'un médicament selon l'une quelconque des revendications 1 à 15, ladite méthode comprenant l'injection ou l'extrusion d'un matériau réservoir dans une gaine creuse.

18. Dispositif de libération intravaginale d'un médicament selon l'une quelconque des revendications 1 à 15 ou méthode selon la revendication 16 ou 17 de fabrication d'un dispositif de libération intravaginale d'un médicament selon l'une quelconque des revendications 1 à 15, dans lequel/laquelle le polymère élastomère hydrophobe est sélectionné parmi des organopolysiloxanes du type pouvant subir une vulcanisation à température ambiante, un poly(éthylène-co-acétate de vinyle) et un copolymère à blocs styrène-butadiène-styrène.
